(19) Europäisches Patentamt
European Patent Office
Office européen des brevets

(11) Veröffentlichungsnummer: **0 333 131 B1**

(12) **EUROPÄISCHE PATENTSCHRIFT**

(45) Veröffentlichungstag der Patentschrift: **27.10.93**

(51) Int. Cl.$^5$: **A01N 25/32**, C07D 231/14

(21) Anmeldenummer: **89104500.7**

(22) Anmeldetag: **14.03.89**

# (54) Pflanzenschützende Mittel auf Basis von Pyrazolcarbonsäurederivaten.

(30) Priorität: **17.03.88 DE 3808896**

(43) Veröffentlichungstag der Anmeldung:
**20.09.89 Patentblatt 89/38**

(45) Bekanntmachung des Hinweises auf die Patenterteilung:
**27.10.93 Patentblatt 93/43**

(84) Benannte Vertragsstaaten:
**AT BE CH DE ES FR GB IT LI**

(56) Entgegenhaltungen:
**EP-A- 0 151 866**
**EP-A- 0 234 119**
**AU-A- 508 225**

**CHEMICAL ABSTRACTS, Band 68, Nr. 19, 6. Mai 1968, Seiten 8421-8422, Nr. 87293y, Columbus, Ohio, US**

(73) Patentinhaber: **HOECHST AKTIENGESELLSCHAFT**

**D-65926 Frankfurt(DE)**

(72) Erfinder: **Sohn, Erich, Dr.**
**Lange Gasse 4**
**D-8900 Augsburg(DE)**
Erfinder: **Mildenberger, Hilmar, Dr.**
**Fasanenstrasse 24**
**D-6233 Kelkheim (Taunus)(DE)**
Erfinder: **Bauer, Klaus Dr.**
**Doorner Strasse 53d**
**D-6450 Hanau(DE)**
Erfinder: **Bieringer, Hermann, Dr.**
**Eichenweg 26**
**D-6239 Eppstein/Taunus(DE)**

EP 0 333 131 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung sind Mittel zum Schutz von Kulturpflanzen gegen phytotoxische Nebenwirkungen von Herbiziden, dadurch gekennzeichnet, daß sie eine Verbindung der Formel I

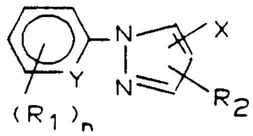

(I),

worin

- Y C-H oder N,
- $R_1$ unabhängig voneinander ($C_1$-$C_4$)-Alkyl, ($C_1$-$C_4$)-Haloalkyl, ($C_1$-$C_4$)-Alkoxy, ($C_1$-$C_4$)-Haloalkoxy oder Halogen,
- $R_2$ ($C_1$-$C_{12}$)-Alkyl oder ($C_3$-$C_7$)-Cycloalkyl,
- X $COOR_3$, $CON(R_4)_2$, $COSR_3$, CN,

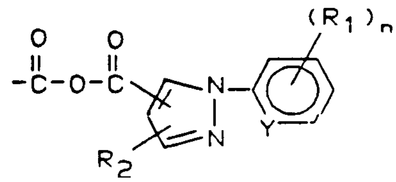

- $R_3$ Alkali- oder Erdalkalimetall, Wasserstoff, ($C_1$-$C_{10}$)-Alkyl, ($C_3$-$C_{20}$)-Alkenyl, ($C_3$-$C_{10}$)-Alkinyl, ($C_3$-$C_7$)-Cycloalkyl, Phenyl-($C_1$-$C_4$)-Alkyl, wobei Phenyl durch Halogen substituiert sein kann, Tris-($C_1$-$C_4$)-Alkyl-Silyl-($C_1$-$C_4$)-Alkyl, ($C_1$-$C_4$)-Alkoxy-($C_1$-$C_4$)-Alkyl
- $R_4$ unabhängig voneinander H, ($C_1$-$C_{10}$)-Alkyl, ($C_3$-$C_7$)-Cycloalkyl, das substituiert sein kann, oder 2 Reste $R_4$ bilden zusammen mit dem sie verknüpfenden N-Atom einen 4- bis 7-gliedrigen heterocyclischen Ring und
- n 1 bis 3

bedeuten, in Kombination mit einem Herbizid enthalten.

Dabei bedeutet Alkyl geradkettiges oder verzweigtes Alkyl.

Im Fall X =

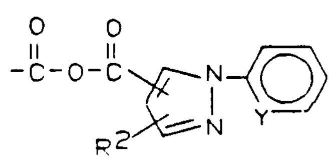

werden zwei identische Reste einer Verbindung der Formel I miteinander verknüpft.

Halogen bedeutet bevorzugt Chlor oder Brom, Alkalimetall bevorzugt Li, Na, K und Erdalkalimetall insbesondere Ca. Bei dem aus den beiden Resten $R_4$ zusammen mit dem N-Atom gebildeten heterocyclischen Ring handelt es sich bevorzugt um Pyrrolidin, Morpholin, 1,2,4-Triazol und Piperidin.

Weiterhin bevorzugt sind die Verbindungen der Formel I, worin Y= CH, $R_1$ = Halogen, ($C_1$-$C_4$)-Haloalkyl, $R_2$ = ($C_1$-$C_6$)-Alkyl, X= $COOR_3$, $R_3$ = H oder ($C_1$-$C_6$)-Alkyl und n= 1 oder 2 bedeuten.

Insbesondere bevorzugt sind die Verbindungen der Formel I, worin Y= CH, $R_1$ = Cl oder Br, $CF_3$, $R_2$ = ($C_1$-$C_4$)-Alkyl, X= $COOR_3$, $R_3$ = ($C_1$-$C_4$)-Alkyl und n= 2 bedeuten.

Die Verbindungen der Formel I mit Y= CH, $R_1$ = 2,4-$Cl_2$, $R_2$ = Isopropyl, X= $COOR_3$ und $R_3$ = ($C_1$-$C_{10}$)-Alkyl sind neu und ebenfalls Gegenstand der vorliegenden Erfindung. Dabei ist für $R_2$ die 5-Stellung und für X die 3-Stellung bevorzugt. Besondere Bedeutung hat die Verbindung mit Y= CH, $R_1$ = 2,4-$Cl_2$, $R_2$ = 5-Isopropyl und X= 3-$COOC_2H_5$.

Die Verbindungen der Formel I lassen sich nach literaturbekannten Methoden herstellen (HU-PS 153 762 od. Chem. Abstr. 68, 87293 y (1968)). Zur weiteren Derivatisierung wird der Rest -$COOR_3$ in bekannter Weise in andere für $\overline{X}$ genannte Reste umgewandelt, z.B. durch Verseifung, Umesterung, Amidierung,

Salzbildung etc., wie dies z.B. in den DE-OS 3 444 918 oder 3 442 690 beschrieben ist.

Bei der Anwendung von Pflanzenbehandlungsmitteln, insbesondere von Herbiziden, können unerwünschte, nicht tolerierbare Schäden an Kulturpflanzen auftreten. Besonders bei der Applikation von Herbiziden nach dem Auflaufen der Kulturpflanzen besteht daher oft das Bedürfnis, das Risiko einer möglichen Phytotoxizität zu vermeiden.

Einige der Verbindungen der Formel I, worin X = (subst.) 4-carboxamid bedeutet, sind bereits aus EP-A-0151866 bekennt. Ihre Safenerwirkung war jedoch bisher nicht erkannt worden.

Aus EP-A-0174 562 sind Safener aus der Reihe der 1-Phenyl-1,3,5-triazol-4-carbonsäurederivate bekennt. Deren Safenerwirkung ist bei einigen Kulturpflanzen jedoch nicht ausreichend.

Verschiedene Verbindungen wurden für diese Anwendung bereits beschrieben (z.B. EP-A 152 006).

Überraschenderweise wurde gefunden, daß Verbindungen der Formel I die Eigenschaften haben, phytotoxische Nebenwirkungen von Pflanzenschutzmitteln, insbesondere von Herbiziden, beim Einsatz in Nutzpflanzenkulturen zu vermindern oder ganz auszuschalten. Die Verbindungen der Formel I sind in der Lage, schädliche Nebenwirkungen der Herbizide völlig aufzuheben, ohne die Wirksamkeit dieser Herbizide gegen Schadpflanzen zu schmälern.

Solche Verbindungen, die die Eigenschaften besitzen, Kulturpflanzen gegen phytotoxische Schäden durch Herbizide zu schützen, ohne die eigentliche herbizide Wirkung dieser Mittel zu beeinträchtigen, werden "Antidote" oder "Safener" genannt.

Das Einsatzgebiet herkömmlicher Herbizide kann durch Zugabe der Safenerverbindung der Formel I ganz erheblich vergrößert werden.

Gegenstand der vorliegenden Erfindung ist daher auch ein Verfahren zum Schutz von Kulturpflanzen gegen phytotoxische Nebenwirkungen von Pflanzenschutzmitteln, insbesondere Herbiziden, das dadurch gekennzeichnet ist, daß man die Pflanzen, Pflanzensamen oder Anbauflächen mit einer Verbindung der Formel I vor, nach oder gleichzeitig mit dem Pflanzenschutzmittel behandelt.

Herbizide, deren phytotoxische Nebenwirkungen mittels der Verbindungen der Formel I herabgesetzt werden können, sind z.B. Carbamate, Thiocarbamate, Halogenacetanilide, substituierte Phenoxy-, Naphthoxy- und Phenoxyphenoxy-carbonsäurederivate sowie Heteroaryloxyphenoxycarbonsäurederivate wie Chinolyloxy-, Chinoxalyloxy, Pyridyloxy-, Benzoxazolyloxy-, Benzthiazolyloxy-phenoxy-carbonsäureester und ferner Dimedonoximabkömmlinge. Bevorzugt hiervon sind Phenoxyphenoxy- und Heteroaryloxyphenoxy-carbonsäureester. Als Ester kommen hierbei insbesondere niedere Alkyl-, Alkenyl- und Alkinylester in Frage.

Beispielsweise seien, ohne daß dadurch eine Beschränkung erfolgen soll, folgende Herbizide genannt:

A) Herbizide vom Typ der Phenoxyphenoxy- und Heteroaryloxyphenoxycarbonsäure-($C_1$-$C_4$)-Alkyl-, ($C_2$-$C_4$)-Alkenyl- oder ($C_3$-$C_4$)-Alkinylester wie
2-(4-(2,4-Dichlorphenoxy)-phenoxy)-propionsäuremethylester,
2-(4-(4-Brom-2-chlorphenoxy)-phenoxy)-propionsäuremethylester,
2-(4-(4-Trifluormethylphenoxy)-phenoxy)-propionsäuremethylester,
2-(4-(2-Chlor-4-trifluormethylphenoxy)-phenoxy)-propionsäuremethylester,
2-(4-(2,4-Dichlorbenzyl)-phenoxy)-propionsäuremethylester,
2-Isopropylideneamino-oxyethyl(R)-2-[4-(6-chloroquinoxalin-2-yloxy)-phenoxy]-propionate (Propaquizafop),
4-(4-(4-Trifluormethylphenoxy)-phenoxy)-pent-2-en-säureethylester,
2-(4-(3,5-Dichlorpyridyl-2-oxy)-phenoxy)-propionsäureethylester,
2-(4-(3,5-Dichlorpyridyl-2-oxy)-phenoxy)-propionsäurepropargylester,
2-(4-(6-Chlorbenzoxazol-2-yl-oxy)-phenoxy)-propionsäureethylester,
2-(4-(6-Chlorbenzthiazol-2-yl-oxy)-phenoxy)-propionsäureethylester,
2-(4-(3-Chlor-5-trifluormethyl-2-pyridyloxy)-phenoxy)-propionsäuremethylester,
2-(4-(5-Trifluormethyl-2-pyridyloxy)-phenoxy)-propionsäurebutylester,
2-(4-(6-Chlor-2-chinoxalyloxy)-phenoxy)-propionsäureethylester,
2-(4-(6-Fluor-2-chinoxalyloxy)-phenoxy)-propionsäureethylester,
2-(4-(5-Chlor-3-fluor-pyridyl-2-oxy)-phenoxy)-propionsäurepropargylester
2-(4-(6-Chlor-2-chinolyloxy)-phenoxy)-propionsäureethylester,
2-(4-(3,5-Dichlorpyridyl-2-oxy)-phenoxy)-propionsäure-trimethylsilylmethylester,
2-(4-(3-Chlor-5-trifluormethoxy-2-pyridyloxy)-phenoxy)-propionsäureethylester,

B) Chloracetanilid-Herbizide wie
N-Methoxymethyl-2,6-diethyl-chloracetanilid,
N-(3′-Methoxyprop-2′-yl)-methyl-6-ethyl-chloracetanilid,
N-(3-Methyl-1,2,4-oxdiazol-5-yl-methyl)-chloressigsäure-2,6-dimethylanilid,

C) Thiocarbamate wie
S-Ethyl-N,N-dipropylthiocarbamat oder
S-Ethyl-N,N-diisobutylthiocarbamat
D) Dimedon-Derivate wie
2-(N-Ethoxybutyrimidoyl)-5-(2-ethylthiopropyl)-3-hydroxy-2-cyclohexen-1-on,
2-(N-Ethoxybutyrimidoyl)-5-(2-phenylthiopropyl)-3-hydroxy-2-cyclohexen-1-on oder
2-(1-Allyloxyiminbutyl)-4-methoxycarbonyl-5,5-dimethyl-3-oxocyclohexenol,
2-(N-Ethoxypropionamidoyl)-5-mesityl-3-hydroxy-2-cyclohexen-1-on,
2-(N-Ethoxybutyrimidoyl)-3-hydroxy-5-(thian-3-yl)-2-cyclohexen-1-on.
2-[1-(Ethoxyimino)-butyl]-3-hydroxy-5-(2H-tetrahydrothiopyran-3-yl)-2-cyclohexen-1-one (BASF 517);
2-[1-(Ethoxyimino)-propyl]-3-hydroxy-5-mesitylcyclohex-2-enone (PP 604 von ICI);
(±)-2-[(E)-3-chloroallyloxyiminopropyl]-5-(2-ethylthiopropyl)-3-hydroxycyclohex-2-enone (Clethodim)

Von den Herbiziden, welche erfindungsgemäß mit den Verbindungen der Formel I kombiniert werden können, sind bevorzugt die unter A) aufgeführten Verbindungen zu nennen, insbesondere 2-(4-(6-Chlorbenzoxazol-2-yl-oxy)-phenoxy)-propionsäureethylester, 2-(4-(6-Chlorbenzthiazol-2-yl-oxy)-phenoxy)-propionsäureethylester und 2-(4-(5-Chlor-3-fluor-pyridyl-2-oxy)-phenoxy)-propionsäurepropargylester. Von den unter D) genannten Substanzen ist insbesondere 2-(N-Ethoxypropionamidoyl)-5-mesityl-3-hydroxy-2-cyclohexen-1-on von Bedeutung.

Das Mengenverhältnis Safener (Verbindung I) : Herbizid kann innerhalb weiter Grenzen zwischen 1 : 10 und 10 : 1, insbesondere zwischen 2 : 1 und 1 : 10 schwanken.

Die jeweils optimalen Mengen an Herbizid und Safener sind abhängig vom Typ des verwendeten Herbizids oder vom verwendeten Safener sowie von der Art des zu behandelnden Pflanzenbestandes und lassen sich von Fall zu Fall durch entsprechende Versuche ermitteln.

Haupteinsatzgebiete für die Anwendung der Safener sind vor allem Getreidekulturen (Weizen, Roggen, Gerste, Hafer), Reis, Mais, Sorghum aber auch Baumwolle, Zuckerrüben, Zuckerrohr und Sojabohne.

Die Safener können je nach ihren Eigenschaften zur Vorbehandlung des Saatgutes der Kulturpflanze (Beizung der Samen) verwendet werden oder vor der Saat in die Saatfurchen eingebracht werden oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein.

Bevorzugt ist jedoch die gleichzeitige Anwendung des Antidots mit dem Herbizid in Form von Tankmischungen oder Fertigformulierungen.

Die Verbindungen der Formel I oder deren Kombination mit einem oder mehreren der genannten Herbizide bzw. Herbizidgruppen können auf verschiedene Art formuliert werden, je nachdem wie es durch die biologischen und/oder chemisch-physikalischen Parameter vorgegeben ist. Als Formulierungsmöglichkeiten kommen daher infrage: Spritzpulver (WP), emulgierbare Konzentrate (EC), wäßrige Lösungen (SC), Emulsionen, versprühbare Lösungen, Dispersionen auf Öl- oder Wasserbasis (SC), Suspoemulsionen (SC), Stäubemittel (DP), Beizmittel, Granulate in Form von Mikro, Sprüh-, Aufzugs- und Adsorptionsgranulaten, wasserdispergierbare Granulate (WG), ULV-Formulierungen, Mikrokapseln oder Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Introduction to Clay Colloid Chemistry", 2nd Ed., J. Wiley & Sons, N.Y.; Marschen, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl-oder Alkylphenolsulfonate und Dispergiermittel, z.B. ligninsulfonsaures Natrium, 2,2′-dinaphthylmethan-6,6′-disulfonsaures Natrium, dibutylnaphthalin-sulfonsaures Natrium oder auch oleylmethyltaurinsaures Natrium enthalten. Emulgierbare Konzentrate werden durch Auflösen des

Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester. Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersion und teilweise und auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids u.a. variiert die erforderliche Aufwandmenge der Verbindungen der Formel I. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Folgende Beispiele dienen zur Erläuterung der Erfindung:

## A. Formulierungsbeispiele

a) Ein Stäubemittel wird erhalten, indem man 10 Gew.-Teile einer Verbindung der Formel I und 90 Gew.-Teile Talkum oder Inertstoff mischt und in einer Schlagmühle zerkleinert.

b) Ein in Wasser leicht dispergierbares, benetzbares Pulver wird enthalten, indem man 25 Gewichtsteile einer Verbindung der Formel I, 64 Gewichtsteile kaolinhaltigen Quarz als Inertstoff, 10 Gewichtsteile lignigsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gewichtsteile einer Verbindung der Formel I mit 6 Gew.-Teilen Alkylphenolpolyglykolether (®Triton X 207), 3 Gew.-Teilen Isotridecanolpolyglykolether (8AeO) und 71 Gew.-Teilen paraffinischem Mineralöl (Siedebereich z. B. ca. 255 bis über 277 °C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel I, 75 Gew.-Teilen Cyclohexanon als Lösungsmittel und 10 Gew.-Teilen oxethyliertes Nonylphenol als Emulgator.

e) Ein in Wasser leicht emulgierbares Konzentrat aus einem Phenoxycarbonsäureester und einem Antidot (10 : 1) wird erhalten aus:

12,00 Gew.-% 2-(4-(6-Chlorbenoxazol-2-yl-oxy)-phenoxy-propionsäureethylester
1,20 Gew.-% Verbindung der Formel I
69,00 Gew.-% Xylol
7,80 Gew.-% dodecylbenzolsulfonsaurem Calcium
6,00 Gew.-% ethoxyliertem Nonylphenol (10 EO)
4,00 Gew.-% ethoxyliertem Rizinusöl (40 EO)

Die Zubereitung erfolgt wie unter Beispiel a) angegeben.

f) Ein in Wasser leicht emulgierbares Konzentrat aus einem Phenoxycarbonsäureester und einem Antidot (1 : 10) wird erhalten aus:

4,0 Gew.-% 2-(4-(6-Chlorbenzoxazol-2-yl-oxy)-phenoxy-propionsäureethylester
40,0 Gew.-% Verbindung der Formel I
30,0 Gew.-% Xylol
20,0 Gew.-% Cyclohexanon
4,0 Gew.-% dodecylbenzolsulfonsaurem Calcium
2,0 Gew.-% ethoxyliertem Rizinusöl (40 EO)

B. Chemische Beispiele

1. 1-(4-Chlorphenyl)-5(3)-methyl-pyrazol-3(5)-carbonsäureethylester

Zu 15,8 g Acetylbrenztraubensäureethylester I in 100 ml Toluol gibt man 14,3 g 4-Chlorphenylhydrazin II und 0,1 g p-Toluolsulfonsäure unter Rühren hinzu und erhitzt am Wasserabscheider. Nachdem kein Wasser mehr übergeht, läßt man abkühlen, verdünnt mit 100 ml Toluol und wäscht mit 100 ml 3 n Salzsäure, 100 ml Wasser, 100 ml gesättigter $NaHCO_3$-Lösung und 100 ml Wasser, engt die organische Phase zur Trockne ein und chromatographiert über Kieselgel (Laufmittel Petrolether → Essigester).

Beisp.Nr.

1 1-(4-Chlorphenyl)-5-methyl-pyrazol-3-carbonsäureethyl ester (Fp. 121-124 °C)

62 1-(4-Chlorphenyl)-3-methyl-pyrazol-5-carbonsäureethylester (Öl)

Analog werden Pyrazole mit anderem Substitutionsmuster im Aromatenteil und/oder anderem Allylrest hergestellt und gegebenenfalls an der Carbonylfunktion derivatisiert. Die Derivate sind in Tabelle I zusammengestellt.

Tabelle I Alkyl-Aryl-pyrazolcarbonsäurederivate

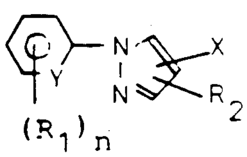

(I)

Y=CH

| Beisp.-Nr. | $(R_1)_n$ | $R_2$ | X | Fp/$Kp_{Torr}$ [°C] |
|---|---|---|---|---|
| 2 | 4-Cl | 5-$CH_3$ | 3-$COOCH_3$ | |
| 3 | " | " | 3-COO-n-$C_3H_7$ | |
| 4 | " | " | 3-COO-i-$C_3H_7$ | |
| 5 | " | " | 3-COO-n-$C_4H_9$ | |
| 6 | " | " | 3-COO-n-$C_5H_{11}$ | |
| 7 | " | " | 3-COO-n-$C_6H_{13}$ | |
| 8 | " | " | 3-COO-n-$C_8H_{17}$ | |
| 9 | " | " | 3-COO-n-$C_{10}H_{21}$ | |
| 10 | " | " | 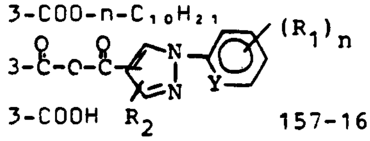 | |
| 11 | " | " | 3-COOH | 157-160 |
| 12 | " | " | 3-COOLi | |
| 13 | " | " | 3-COONa | |
| 14 | " | " | 3-COOK | |
| 15 | " | " | 3-$COOCa_{1/2}$ | |
| 16 | " | " | 3-COO-c-$C_4H_7$ | |
| 17 | " | " | 3-COO-c-$C_6H_{13}$ | |
| 18 | " | " | 3-$COOCH_2$-$C_6H_5$ | |
| 19 | " | " | 3-$COOCH_2$-(2,4-$Cl_2$-$C_6H_3$) | |
| 20 | " | " | 3-$COOCH_2CHCH_2$ | |
| 21 | " | " | 3-$COOC_2H_4CHCH_2$ | |
| 22 | " | " | 3-COO-n-$C_8H_{16}CHCH_2$ | |
| 23 | " | " | 3-$COOCH_2CCH$ | |
| 24 | " | " | 3-COO-$C_2H_4$-CCH | |
| 25 | " | " | 3-COO-n-$C_5H_{10}CCH$ | |
| 26 | " | " | 3-$COOCH_2Si(CH_3)_3$ | |
| 27 | " | " | 3-$COOC_2H_4OCH_3$ | |
| 28 | " | " | 3-$CONH_2$ | |
| 29 | " | " | 3-CN | |
| 30 | " | " | 3-$CONHCH_3$ | |

Y=CH

| Beisp.-Nr. | $(R_1)_n$ | $R_2$ | X | $Fp/Kp_{Torr}$ [°C] |
|---|---|---|---|---|
| 31 | 4-Cl | 5-$CH_3$ | 3-$CONHC_2H_5$ | |
| 32 | " | " | 3-CONH-n-$C_3H_7$ | |
| 33 | " | " | 3-CONH-n-$C_4H_9$ | |
| 34 | " | " | 3-CONH-n-$C_6H_{13}$ | |
| 35 | " | " | 3-CONH-n-$C_{10}H_{21}$ | |
| 36 | " | " | 3-CONH-i-$C_3H_7$ | |
| 37 | " | " | 3-$CON(CH_3)_2$ | |
| 38 | " | " | 3-$CON(CH_3)(nC_6H_{13})$ | |
| 39 | " | " | 3-$CON(C_2H_5)_2$ | |
| 40 | " | " | 3-CO-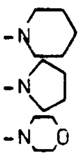 | |
| 41 | " | " | 3-CO- | |
| 42 | " | " | 3-CO- | |
| 43 | " | " | 3-CO-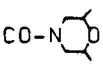 | |
| 44 | " | " | 3-CO-NH-c-$C_6H_{11}$ | |
| 45 | " | " | 3-CO-NH-c-$C_3H_5$ | |
| 46 | " | " | 3-CO-$N(CH_3)(cC_6H_{11})$ | |
| 47 | " | " | 3-COSH | |
| 48 | " | " | 3-COSNa | |
| 49 | " | " | 3-$COSCH_3$ | |
| 50 | " | " | 3-$COSC_2H_5$ | |
| 51 | " | " | 3-$COSCH_2C_6H_5$ | |
| 52 | " | " | 3-COS-n$C_8H_{17}$ | |
| 53 | " | " | 3-$COSC_2H_4OCH_3$ | |
| 54 | " | " | 3-$COSCH_2CHCH_2$ | |
| 55 | " | " | 3-$COSCH_2CCH$ | |
| 56 | " | " | 3-COS-c-$C_6H_{11}$ | |
| 57 | " | " | 3-$COSCH_2Si(CH_3)_3$ | |
| 58 | " | " | 3-COS-n-$C_4H_8CH(CH_3)_2$ | |
| 59 | " | " | 3-CO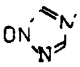 | |
| 60 | " | " | 3-$COOC_2H_4CH(CH_3)_2$ | |

Y=CH

| Beisp.-Nr. | $(R_1)_n$ | $R_2$ | X | Fp/$Kp_{Torr}$ [°C] |
|---|---|---|---|---|
| 61 | 4-Cl | 3-$CH_3$ | 5-$COOCH_3$ | |
| 63 | " | " | 5-$COOnC_3H_7$ | |
| 64 | " | " | 5-COO-i-$C_3H_7$ | |
| 65 | " | " | 5-COO-n-$C_4H_9$ | |
| 66 | " | " | 5-COO-n-$C_5H_{11}$ | |
| 67 | " | " | 5-COO-n-$C_6H_{13}$ | |
| 68 | " | " | 5-COO-n-$C_8H_{17}$ | |
| 69 | " | " | 5-COO-n-$C_{10}H_{21}$ | |
| 70 | " | " | 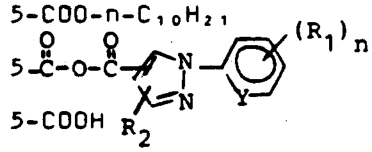 | |
| 71 | " | " | 5-COOH | |
| 72 | " | " | 5-COOLi | |
| 73 | " | " | 5-COONa | |
| 74 | " | " | 5-COOK | |
| 75 | " | " | 5-$COOCa_{1/2}$ | |
| 76 | " | " | 5-COO-c-$C_4H_7$ | |
| 77 | " | " | 5-COO-c-$C_6H_{11}$ | |
| 78 | " | " | 5-$COOCH_2$-$C_6H_5$ | |
| 79 | " | " | 5-$COOCH_2$-(2,4-$Cl_2$-$C_6H_3$) | |
| 80 | " | " | 5-$COOCH_2CHCH_2$ | |
| 81 | " | " | 5-$COOC_2H_4CHCH_2$ | |
| 82 | " | " | 5-COO-n-$C_8H_{16}CHCH_2$ | |
| 83 | " | " | 5-COO-$CH_2CCH$ | |
| 84 | " | " | 5-COO-$C_2H_4$-CCH | |
| 85 | " | " | 5-COO-n-$C_5H_{10}CCH$ | |
| 86 | " | " | 5-$COOCH_2Si(CH_3)_3$ | |
| 87 | " | " | 5-$COOC_2H_4OCH_3$ | |
| 88 | " | " | 5-$CONH_2$ | |
| 89 | " | " | 5-CN | |
| 90 | " | " | 5-$CONHCH_3$ | |

| Y=CH Beisp.-Nr. | $(R_1)_n$ | $R_2$ | X | $Fp/Kp_{Torr}$ [°C] |
|---|---|---|---|---|
| 91 | 4-Cl | 3-$CH_3$ | 5-$CONHC_2H_5$ | |
| 92 | " | " | 5-CONH-n-$C_3H_7$ | |
| 93 | " | " | 5-CONH-n-$C_4H_9$ | |
| 94 | " | " | 5-CONH-n-$C_6H_{13}$ | |
| 95 | " | " | 5-CONH-n-$C_{10}H_{21}$ | |
| 96 | " | " | 5-CONH-i-$C_3H_7$ | |
| 97 | " | " | 5-$CON(CH_3)_2$ | |
| 98 | " | " | 5-$CON(CH_3)(nC_6H_{13})$ | |
| 99 | " | " | 5-$CON(C_2H_5)_2$ | |
| 100 | " | " | 5-CO-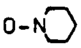 | |
| 101 | " | " | 5-CO-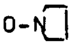 | |
| 102 | " | " | 5-CO-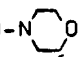 | |
| 103 | " | " | 5-CO-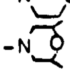 | |
| 104 | " | " | 5-CO-NH-c-$C_6H_{11}$ | |
| 105 | " | " | 5-CO-NH-c-$C_3H_5$ | |
| 106 | " | " | 5-CO-$N(CH_3)(cC_6H_{11})$ | |
| 107 | " | " | 5-COSH | |
| 108 | " | " | 5-COSNa | |
| 109 | " | " | 5-$COSCH_3$ | |
| 110 | " | " | 5-$COSC_2H_5$ | |
| 111 | " | " | 5-$COSCH_2C_6H_5$ | |
| 112 | " | " | 5-COS-$nC_8H_{17}$ | |
| 113 | " | " | 5-$COSC_2H_4OCH_3$ | |
| 114 | " | " | 5-$COSCH_2CHCH_2$ | |
| 115 | " | " | 5-$COSCH_2CCH$ | |
| 116 | " | " | 5-COS-c-$C_6H_{11}$ | |
| 117 | " | " | 5-$COSCH_2Si(CH_3)_3$ | |
| 118 | " | " | 5-COS-n-$C_4H_8CH(CH_3)_2$ | |
| 119 | " | " | 5-CO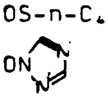 | |
| 120 | " | " | 5-$COOC_2H_4CH(CH_3)_2$ | |

Y=CH

| Beisp.-Nr. | $(R_1)_n$ | $R_2$ | X | $Fp/Kp_{Torr}$ [°C] |
|---|---|---|---|---|
| 121 | 2,4-$Cl_2$ | 5-$CH_3$ | 3-$COOCH_3$ | 87-93 |
| 122 | " | " | 3-$COOC_2H_5$ | 76-81 |
| 123 | " | " | 3-COO-n-$C_3H_7$ | 99-100 |
| 124 | " | " | 3-COO-i-$C_3H_7$ | 65-70 |
| 125 | " | " | 3-COO-n-$C_4H_9$ | 76-78 |
| 126 | " | " | 3-COO-n-$C_5H_{11}$ | |
| 127 | " | " | 3-COO-n-$C_6H_{13}$ | Öl |
| 128 | " | " | 3-COO-n-$C_8H_{17}$ | 47-49 |
| 129 | " | " | 3-COO-n-$C_{10}H_{21}$ | |
| 130 | " | " | 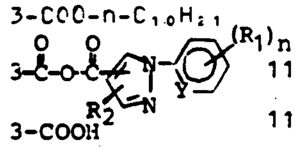 | 114-117 |
| 131 | " | " | 3-COOH | 112-115 |
| 132 | " | " | 3-COOLi | >250 |
| 133 | " | " | 3-COONa | >250 |
| 134 | " | " | 3-COOK | |
| 135 | " | " | 3-$COOCa_{1/2}$ | 187-188 |
| 136 | " | " | 3-COO-c-$C_4H_7$ | |
| 137 | " | " | 3-COO-c-$C_6H_{11}$ | 72-74 |
| 138 | " | " | 3-$COOCH_2$-$C_6H_5$ | Öl |
| 139 | " | " | 3-$COOCH_2$-(2,4-$Cl_2$-$C_6H_3$) | |
| 140 | " | " | 3-$COOCH_2CHCH_2$ | Öl |
| 141 | " | " | 3-$COOC_2H_4CHCH_2$ | |
| 142 | " | " | 3-COO-n-$C_8H_{16}CHCH_2$ | |
| 143 | " | " | 3-COO-$CH_2CCH$ | 101-102 |
| 144 | " | " | 3-COO-$C_2H_4$-CCH | |
| 145 | " | " | 3-COO-n-$C_5H_{10}CCH$ | |
| 146 | " | " | 3-$COOCH_2Si(CH_3)_3$ | 67-70 |
| 147 | " | " | 3-$COOC_2H_4OCH_3$ | Öl |
| 148 | " | " | 3-$CONH_2$ | 161 |
| 149 | " | " | 3-CN | |
| 150 | " | " | 3-$CONHCH_3$ | 161-162 |

Y=CH

| Beisp.-Nr. | $(R_1)_n$ | $R_2$ | X | $Fp/Kp_{Torr}$ [°C] |
|---|---|---|---|---|
| 151 | 2,4-$Cl_2$ | 5-$CH_3$ | 3-$CONHC_2H_5$ | 87-90 |
| 152 | " | " | 3-CONH-n-$C_3H_7$ | 89-92 |
| 153 | " | " | 3-CONH-n-$C_4H_9$ | 55-60 |
| 154 | " | " | 3-CONH-n-$C_6H_{13}$ | 68-71 |
| 155 | " | " | 3-CONH-n-$C_{10}H_{21}$ | |
| 156 | " | " | 3-CONH-i-$C_3H_7$ | |
| 157 | " | " | 3-$CON(CH_3)_2$ | 99-103 |
| 158 | " | " | 3-$CON(CH_3)(nC_6H_{13})$ | |
| 159 | " | " | 3-$CON(C_2H_5)_2$ | Öl |
| 160 | " | " | 3-CO-N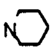 | Harz |
| 161 | " | " | 3-CO-N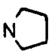 | |
| 162 | " | " | 3-CO-N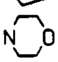 | Öl |
| 163 | " | " | 3-CO-N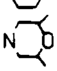 | Harz |
| 164 | " | " | 3-CO-NH-c-$C_6H_{11}$ | 120-122 |
| 165 | " | " | 3-CO-NH-c-$C_3H_5$ | |
| 166 | " | " | 3-CO-$N(CH_3)(cC_6H_{11})$ | Öl |
| 167 | " | " | 3-COSH | |
| 168 | " | " | 3-COSNa | |
| 169 | " | " | 3-$COSCH_3$ | |
| 170 | " | " | 3-$COSC_2H_5$ | |
| 171 | " | " | 3-$COSCH_2C_6H_5$ | 70-73 |
| 172 | " | " | 3-COS-$nC_8H_{17}$ | |
| 173 | " | " | 3-$COSC_2H_4OCH_3$ | |
| 174 | " | " | 3-$COSCH_2CHCH_2$ | |
| 175 | " | " | 3-$COSCH_2CCH$ | |
| 176 | " | " | 3-COS-c-$C_6H_{11}$ | |
| 177 | " | " | 3-$COSCH_2Si(CH_3)_3$ | |
| 178 | " | " | 3-COS-n-$C_4H_8CH(CH_3)_2$ | |
| 179 | " | " | 3-CON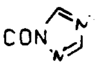 | |
| 180 | " | " | 3-$COOC_2H_4CH(CH_3)_2$ | |

Y=CH

| Beisp.-Nr. | $(R_1)_n$ | $R_2$ | X | Fp/$Kp_{Torr}$ [°C] |
|---|---|---|---|---|
| 181 | 2,4-$Cl_2$ | 3-$CH_3$ | 5-$COOCH_3$ | |
| 182 | " | " | 5-$COOC_2H_5$ | Öl |
| 183 | " | " | 5-COO-n-$C_3H_7$ | |
| 184 | " | " | 5-COO-i-$C_3H_7$ | |
| 185 | " | " | 5-COO-n-$C_4H_9$ | |
| 186 | " | " | 5-COO-n-$C_5H_{11}$ | |
| 187 | " | " | 5-COO-n-$C_6H_{13}$ | |
| 188 | " | " | 5-COO-n-$C_8H_{17}$ | |
| 189 | " | " | 5-COO-n-$C_{10}H_{21}$ | |
| 190 | " | " | 5-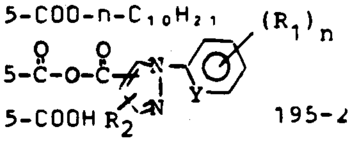 | |
| 191 | " | " | 5-COOH | 195-205 |
| 192 | " | " | 5-COOLi | |
| 193 | " | " | 5-COONa | |
| 194 | " | " | 5-COOK | |
| 195 | " | " | 5-$COOCa_{1/2}$ | |
| 196 | " | " | 5-COO-c-$C_4H_7$ | |
| 197 | " | " | 5-COO-c-$C_6H_{11}$ | |
| 198 | " | " | 5-$COOCH_2$-$C_6H_5$ | |
| 199 | " | " | 5-$COOCH_2$-(2,4-$Cl_2$-$C_6H_3$) | |
| 200 | " | " | 5-$COOCH_2CHCH_2$ | |
| 201 | " | " | 5-$COOC_2H_4CHCH_2$ | |
| 202 | " | " | 5-COO-n-$C_8H_{16}CHCH_2$ | |
| 203 | " | " | 5-COO-$CH_2CCH$ | |
| 204 | " | " | 5-COO-$C_2H_4$-CCH | |
| 205 | " | " | 5-COO-n-$C_5H_{10}CCH$ | |
| 206 | " | " | 5-$COOCH_2Si(CH_3)_3$ | |
| 207 | " | " | 5-$COOC_2H_4OCH_3$ | |
| 208 | " | " | 5-$CONH_2$ | |
| 209 | " | " | 5-CN | |
| 210 | " | " | 5-$CONHCH_3$ | |

Y=CH

| Beisp.-Nr. | $(R_1)_n$ | $R_2$ | X | $Fp/Kp_{Torr}$ [°C] |
|---|---|---|---|---|
| 211 | 2,4-$Cl_2$ | 3-$CH_3$ | 5-$CONHC_2H_5$ | |
| 212 | " | " | 5-CONH-n-$C_3H_7$ | 81 |
| 213 | " | " | 5-CONH-n-$C_4H_9$ | |
| 214 | " | " | 5-CONH-n-$C_6H_{13}$ | |
| 215 | " | " | 5-CONH-n-$C_{10}H_{21}$ | |
| 216 | " | " | 5-CONH-i-$C_3H_7$ | |
| 217 | " | " | 5-$CON(CH_3)_2$ | |
| 218 | " | " | 5-$CON(CH_3)(nC_6H_{13})$ | |
| 219 | " | " | 5-$CON(C_2H_5)_2$ | |
| 220 | " | " | 5-CO-N 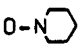 | |
| 221 | " | " | 5-CO-N 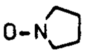 | |
| 222 | " | " | 5-CO-N 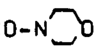 | |
| 223 | " | " | 5-CO-N 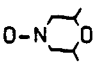 | |
| 224 | " | " | 5-CO-NH-c-$C_6H_{11}$ | |
| 225 | " | " | 5-CO-NH-c-$C_3H_5$ | |
| 226 | " | " | 5-CO-$N(CH_3)(cC_6H_{11})$ | |
| 227 | " | " | 5-COSH | |
| 228 | " | " | 5-COSNa | |
| 229 | " | " | 5-$COSCH_3$ | |
| 230 | " | " | 5-$COSC_2H_5$ | |
| 231 | " | " | 5-$COSCH_2C_6H_5$ | |
| 232 | " | " | 5-COS-n$C_8H_{17}$ | |
| 233 | " | " | 5-$COSC_2H_4OCH_3$ | |
| 234 | " | " | 5-$COSCH_2CHCH_2$ | |
| 235 | " | " | 5-$COSCH_2CCH$ | |
| 236 | " | " | 5-COS-c-$C_6H_{11}$ | |
| 237 | " | " | 5-$COSCH_2Si(CH_3)_3$ | |
| 238 | " | " | 5-COS-n-$C_4H_8CH(CH_3)_2$ | |
| 239 | " | " | 5-CON 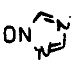 | |
| 240 | " | " | 5-$COOC_2H_4CH(CH_3)_2$ | |

Y=CH

| Beisp.-Nr. | $(R_1)_n$ | $R_2$ | X | $Fp/Kp_{Torr}$ [°C] |
|---|---|---|---|---|
| 241 | 2,4-$Cl_2$ | 5-$C_2H_5$ | 3-$COOCH_3$ | |
| 242 | " | " | 3-$COOC_2H_5$ | 48-49 |
| 243 | " | " | 3-COO-n-$C_3H_7$ | |
| 244 | " | " | 3-COO-i-$C_3H_7$ | |
| 245 | " | " | 3-COO-n-$C_4H_9$ | |
| 246 | " | " | 3-COO-n-$C_5H_{11}$ | |
| 247 | " | " | 3-COO-n-$C_6H_{13}$ | |
| 248 | " | " | 3-COO-n-$C_8H_{17}$ | |
| 249 | " | " | 3-COO-n-$C_{10}H_{21}$ | |
| 250 | " | " | 3-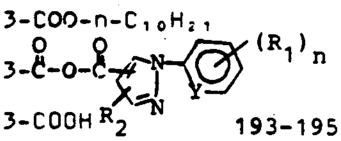 | |
| 251 | " | " | 3-COOH | 193-195 |
| 252 | " | " | 3-COOLi | |
| 253 | " | " | 3-COONa | |
| 254 | " | " | 3-COOK | |
| 255 | " | " | 3-$COOCa_{1/2}$ | |
| 256 | " | " | 3-COO-c-$C_4H_7$ | |
| 257 | " | " | 3-COO-c-$C_6H_{11}$ | |
| 258 | " | " | 3-$COOCH_2$-$C_6H_5$ | |
| 259 | " | " | 3-$COOCH_2$-(2,4-$Cl_2$-$C_6H_3$) | |
| 260 | " | " | 3-$COOCH_2CHCH_2$ | |
| 261 | " | " | 3-$COOC_2H_4CHCH_2$ | |
| 262 | " | " | 3-COO-n-$C_8H_{16}CHCH_2$ | |
| 263 | " | " | 3-COO-$CH_2CCH$ | |
| 264 | " | " | 3-COO-$C_2H_4$-CCH | |
| 265 | " | " | 3-COO-n-$C_5H_{10}CCH$ | |
| 266 | " | " | 3-$COOCH_2Si(CH_3)_3$ | |
| 267 | " | " | 3-$COOC_2H_4OCH_3$ | |
| 268 | " | " | 3-$CONH_2$ | |
| 269 | " | " | 3-CN | |
| 270 | " | " | 3-$CONHCH_3$ | |

Y=CH

| Beisp.-Nr. | $(R_1)_n$ | $R_2$ | X | $Fp/Kp_{Torr}$ [°C] |
|---|---|---|---|---|
| 271 | 2,4-$Cl_2$ | 5-$C_2H_5$ | 3-$CONHC_2H_5$ | |
| 272 | " | " | 3-CONH-n-$C_3H_7$ | |
| 273 | " | " | 3-CONH-n-$C_4H_9$ | |
| 274 | " | " | 3-CONH-n-$C_6H_{13}$ | |
| 275 | " | " | 3-CONH-n-$C_{10}H_{21}$ | |
| 276 | " | " | 3-CONH-i-$C_3H_7$ | |
| 277 | " | " | 3-$CON(CH_3)_2$ | |
| 278 | " | " | 3-$CON(CH_3)(nC_6H_{13})$ | |
| 279 | " | " | 3-$CON(C_2H_5)_2$ | |
| 280 | " | " | 3-CO- 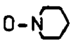 | |
| 281 | " | " | 3-CO- 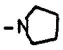 | |
| 282 | " | " | 3-CO- 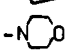 | |
| 283 | " | " | 3-CO- 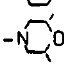 | |
| 284 | " | " | 3-CO-NH-c-$C_6H_{11}$ | |
| 285 | " | " | 3-CO-NH-c-$C_3H_5$ | |
| 286 | " | " | 3-CO-$N(CH_3)(cC_6H_{11})$ | |
| 287 | " | " | 3-COSH | |
| 288 | " | " | 3-COSNa | |
| 289 | " | " | 3-$COSCH_3$ | |
| 290 | " | " | 3-$COSC_2H_5$ | |
| 291 | " | " | 3-$COSCH_2C_6H_5$ | |
| 292 | " | " | 3-COS-n$C_8H_{17}$ | |
| 293 | " | " | 3-$COSC_2H_4OCH_3$ | |
| 294 | " | " | 3-$COSCH_2CHCH_2$ | |
| 295 | " | " | 3-$COSCH_2CCH$ | |
| 296 | " | " | 3-COS-c-$C_6H_{11}$ | |
| 297 | " | " | 3-$COSCH_2Si(CH_3)_3$ | |
| 298. | " | " | 3-COS-n-$C_4H_8CH(CH_3)_2$ | |
| 299 | " | " | 3-CO 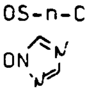 | |
| 300 | " | " | 3-$COOC_2H_4CH(CH_3)_2$ | |

Y=CH

| Beisp.-Nr. | $(R_1)_n$ | $R_2$ | X | Fp/$Kp_{Torr}$ [°C] |
|---|---|---|---|---|
| 301 | 2,4-$Cl_2$ | 5-$CH(CH_3)_2$ | 3-$COOCH_3$ | 144 |
| 302 | " | " | 3-$COOC_2H_5$ | 70-77 |
| 303 | " | " | 3-COO-n-$C_3H_7$ | Öl |
| 304 | " | " | 3-COO-i-$C_3H_7$ | Öl |
| 305 | " | " | 3-COO-n-$C_4H_9$ | |
| 306 | " | " | 3-COO-n-$C_5H_{11}$ | |
| 307 | " | " | 3-COO-n-$C_6H_{13}$ | |
| 308 | " | " | 3-COO-n-$C_8H_{17}$ | |
| 309 | " | " | 3-COO-n-$C_{10}H_{21}$ | |
| 310 | " | " | 3- 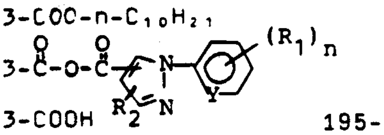 | |
| 311 | " | " | 3-COOH | 195-196 |
| 312 | " | " | 3-COOLi | |
| 313 | " | " | 3-COONa | >250 |
| 314 | " | " | 3-COOK | |
| 315 | " | " | 3-$COOCa_{1/2}$ | |
| 316 | " | " | 3-COO-c-$C_4H_7$ | |
| 317 | " | " | 3-COO-c-$C_6H_{11}$ | |
| 318 | " | " | 3-$COOCH_2$-$C_6H_5$ | |
| 319 | " | " | 3-$COOCH_2$-(2,4-$Cl_2$-$C_6H_3$) | |
| 320 | " | " | 3-$COOCH_2CHCH_2$ | |
| 321 | " | " | 3-$COOC_2H_4CHCH_2$ | |
| 322 | " | " | 3-COO-n-$C_8H_{16}CHCH_2$ | |
| 323 | " | " | 3-COO-$CH_2CCH$ | |
| 324 | " | " | 3-COO-$C_2H_4$-CCH | |
| 325 | " | " | 3-COO-n-$C_5H_{10}CCH$ | |
| 326 | " | " | 3-$COOCH_2Si(CH_3)_3$ | |
| 327 | " | " | 3-$COOC_2H_4OCH_3$ | |
| 328 | " | " | 3-$CONH_2$ | |
| 329 | " | " | 3-CN | |
| 330 | " | " | 3-$CONHCH_3$ | |

Y=CH

| Beisp.-Nr. | $(R_1)_n$ | $R_2$ | X | Fp/$Kp_{Torr}$ [°C] |
|---|---|---|---|---|
| 331 | 2,4-$Cl_2$ | 5-$CH(CH_3)_2$ | 3-$CONHC_2H_5$ | 106-109 |
| 332 | " | " | 3-CONH-n-$C_3H_7$ | 67 |
| 333 | " | " | 3-CONH-n-$C_4H_9$ | |
| 334 | " | " | 3-CONH-n-$C_6H_{13}$ | |
| 335 | " | " | 3-CONH-n-$C_{10}H_{21}$ | |
| 336 | " | " | 3-CONH-i-$C_3H_7$ | |
| 337 | " | " | 3-$CON(CH_3)_2$ | |
| 338 | " | " | 3-$CON(CH_3)(nC_6H_{13})$ | |
| 339 | " | " | 3-$CON(C_2H_5)_2$ | 98-100 |
| 340 | " | " | 3-CO-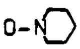 | |
| 341 | " | " | 3-CO-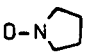 | |
| 342 | " | " | 3-CO-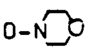 | |
| 343 | " | " | 3-CO-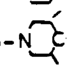 | 140-142 |
| 344 | " | " | 3-CO-NH-c-$C_6H_{11}$ | |
| 345 | " | " | 3-CO-NH-c-$C_3H_5$ | |
| 346 | " | " | 3-CO-$N(CH_3)(cC_6H_{11})$ | |
| 347 | " | " | 3-COSH | |
| 348 | " | " | 3-COSNa | |
| 349 | " | " | 3-$COSCH_3$ | |
| 350 | " | " | 3-$COSC_2H_5$ | |
| 351 | " | " | 3-$COSCH_2C_6H_5$ | |
| 352 | " | " | 3-COS-$nC_8H_{17}$ | |
| 353 | " | " | 3-$COSC_2H_4OCH_3$ | |
| 354 | " | " | 3-$COSCH_2CHCH_2$ | |
| 355 | " | " | 3-$COSCH_2CCH$ | |
| 356 | " | " | 3-COS-c-$C_6H_{11}$ | |
| 357 | " | " | 3-$COSCH_2Si(CH_3)_3$ | |
| 358 | " | " | 3-COS-n-$C_4H_8CH(CH_3)_2$ | |
| 359 | " | " | 3-CO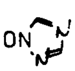 | |
| 360 | " | " | 3-$COOC_2H_4CH(CH_3)_2$ | |

Y=CH

| Beisp.-Nr. | $(R_1)_n$ | $R_2$ | X | $Fp/Kp_{Torr}$ [°C] |
|---|---|---|---|---|
| 361 | 2,4-$Cl_2$ | 5-$C(CH_3)_3$ | 3-$COOCH_3$ | Harz |
| 362 | " | " | 3-$COOC_2H_5$ | 118-121 |
| 363 | " | " | 3-COO-n-$C_3H_7$ | |
| 364 | " | " | 3-COO-i-$C_3H_7$ | |
| 365 | " | " | 3-COO-n-$C_4H_9$ | |
| 366 | " | " | 3-COO-n-$C_5H_{11}$ | |
| 367 | " | " | 3-COO-n-$C_6H_{13}$ | |
| 368 | " | " | 3-COO-n-$C_8H_{17}$ | |
| 369 | " | " | 3-COO-n-$C_{10}H_{21}$ | |
| 370 | " | " | 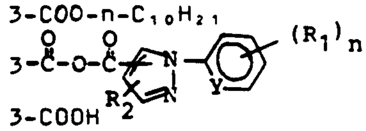 | |
| 371 | " | " | 3-COOH | |
| 372 | " | " | 3-COOLi | |
| 373 | " | " | 3-COONa | |
| 374 | " | " | 3-COOK | |
| 375 | " | " | 3-$COOCa_{1/2}$ | |
| 376 | " | " | 3-COO-c-$C_4H_7$ | |
| 377 | " | " | 3-COO-c-$C_6H_{11}$ | |
| 378 | " | " | 3-$COOCH_2$-$C_6H_5$ | |
| 379 | " | " | 3-$COOCH_2$-(2,4-$Cl_2$-$C_6H_3$) | |
| 380 | " | " | 3-$COOCH_2CHCH_2$ | |
| 381 | " | " | 3-$COOC_2H_4CHCH_2$ | |
| 382 | " | " | 3-COO-n-$C_8H_{16}CHCH_2$ | |
| 383 | " | " | 3-COO-$CH_2CCH$ | |
| 384 | " | " | 3-COO-$C_2H_4$-CCH | |
| 385 | " | " | 3-COO-n-$C_5H_{10}CCH$ | |
| 386 | " | " | 3-$COOCH_2Si(CH_3)_3$ | |
| 387 | " | " | 3-$COOC_2H_4OCH_3$ | |
| 388 | " | " | 3-$CONH_2$ | |
| 389 | " | " | 3-CN | |
| 390 | " | " | 3-$CONHCH_3$ | |

Y=CH

| Beisp.-Nr. | $(R_1)_n$ | $R_2$ | X | $Fp/Kp_{Torr}$ [°C] |
|---|---|---|---|---|
| 391 | 2,4-$Cl_2$ | 5-$C(CH_3)_3$ | 3-$CONHC_2H_5$ | 161-162 |
| 392 | " | " | 3-CONH-n-$C_3H_7$ | 102-103 |
| 393 | " | " | 3-CONH-n-$C_4H_9$ | |
| 394 | " | " | 3-CONH-n-$C_6H_{13}$ | |
| 395 | " | " | 3-CONH-n-$C_{10}H_{21}$ | |
| 396 | " | " | 3-CONH-i-$C_3H_7$ | |
| 397 | " | " | 3-$CON(CH_3)_2$ | |
| 398 | " | " | 3-$CON(CH_3)(nC_6H_{13})$ | |
| 399 | " | " | 3-$CON(C_2H_5)_2$ | |
| 400 | " | " | 3-CO-N 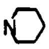 | |
| 401 | " | " | 3-CO-N 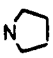 | |
| 402 | " | " | 3-CO-N 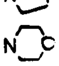 | |
| 403 | " | " | 3-CO-N 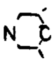 | |
| 404 | " | " | 3-CO-NH-c-$C_6H_{11}$ | |
| 405 | " | " | 3-CO-NH-c-$C_3H_5$ | |
| 406 | " | " | 3-CO-$N(CH_3)(cC_6H_{11})$ | |
| 407 | " | " | 3-COSH | |
| 408 | " | " | 3-COSNa | |
| 409 | " | " | 3-$COSCH_3$ | |
| 410 | " | " | 3-$COSC_2H_5$ | |
| 411 | " | " | 3-$COSCH_2C_6H_5$ | |
| 412 | " | " | 3-COS-$nC_8H_{17}$ | |
| 413 | " | " | 3-$COSC_2H_4OCH_3$ | |
| 414 | " | " | 3-$COSCH_2CHCH_2$ | |
| 415 | " | " | 3-$COSCH_2CCH$ | |
| 416 | " | " | 3-COS-c-$C_6H_{11}$ | |
| 417 | " | " | 3-$COSCH_2Si(CH_3)_3$ | |
| 418 | " | " | 3-COS-n-$C_4H_8CH(CH_3)_2$ | |
| 419 | " | " | 3-CON 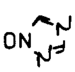 | |
| 420 | " | " | 3-$COOC_2H_4CH(CH_3)_2$ | |

Y=CH

| Beisp.-Nr. | $(R_1)_n$ | $R_2$ | X | Fp/Kp$_{Torr}$ [°C] |
|---|---|---|---|---|
| 421 | 2,4-$Cl_2$ | 5-$CH_2$-$CH(CH_3)_2$ | 3-$COOCH_3$ | |
| 422 | " | " | 3-$COOC_2H_5$ | Öl |
| 423 | " | " | 3-COO-n-$C_3H_7$ | |
| 424 | " | " | 3-COO-i-$C_3H_7$ | |
| 425 | " | " | 3-COO-n-$C_4H_9$ | |
| 426 | " | " | 3-COO-n-$C_5H_{11}$ | |
| 427 | " | " | 3-COO-n-$C_6H_{13}$ | |
| 428 | " | " | 3-COO-n-$C_8H_{17}$ | |
| 429 | " | " | 3-COO-n-$C_{10}H_{21}$ | |
| 430 | " | " | 3-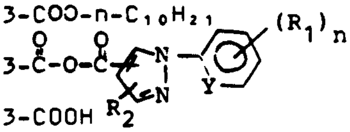 | |
| 431 | " | " | 3-COOH | |
| 432 | " | " | 3-COOLi | |
| 433 | " | " | 3-COONa | |
| 434 | " | " | 3-COOK | |
| 435 | " | " | 3-$COOCa_{1/2}$ | |
| 436 | " | " | 3-COO-c-$C_4H_7$ | |
| 437 | " | " | 3-COO-c-$C_6H_{11}$ | |
| 438 | " | " | 3-$COOCH_2$-$C_6H_5$ | |
| 439 | " | " | 3-$COOCH_2$-(2,4-$Cl_2$-$C_6H_3$) | |
| 440 | " | " | 3-$COOCH_2CHCH_2$ | |
| 441 | " | " | 3-$COOC_2H_4CHCH_2$ | |
| 442 | " | " | 3-COO-n-$C_8H_{16}CHCH_2$ | |
| 443 | " | " | 3-COO-$CH_2CCH$ | |
| 444 | " | " | 3-COO-$C_2H_4$-CCH | |
| 445 | " | " | 3-COO-n-$C_5H_{10}CCH$ | |
| 446 | " | " | 3-$COOCH_2Si(CH_3)_3$ | |
| 447 | " | " | 3-$COOC_2H_4OCH_3$ | |
| 448 | " | " | 3-$CONH_2$ | |
| 449 | " | " | 3-CN | |
| 450 | " | " | 3-$CONHCH_3$ | |

Y=CH

| Beisp.-Nr. | $(R_1)_n$ | $R_2$ | X | Fp/Kp$_{Torr}$ [°C] |
|---|---|---|---|---|
| 451 | 2,4-$Cl_2$ | 5-$CH_2CH(CH_3)_3$ | 3-$CONHC_2H_5$ | |
| 452 | " | " | 3-CONH-n-$C_3H_7$ | |
| 453 | " | " | 3-CONH-n-$C_4H_9$ | |
| 454 | " | " | 3-CONH-n-$C_6H_{13}$ | |
| 455 | " | " | 3-CONH-n-$C_{10}H_{21}$ | |
| 456 | " | " | 3-CONH-i-$C_3H_7$ | |
| 457 | " | " | 3-$CON(CH_3)_2$ | |
| 458 | " | " | 3-$CON(CH_3)(nC_6H_{13})$ | |
| 459 | " | " | 3-$CON(C_2H_5)_2$ | |
| 460 | " | " | 3-CO-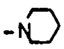 | |
| 461 | " | " | 3-CO-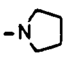 | |
| 462 | " | " | 3-CO-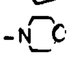 | |
| 463 | " | " | 3-CO-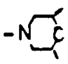 | |
| 464 | " | " | 3-CO-NH-c-$C_6H_{11}$ | |
| 465 | " | " | 3-CO-NH-c-$C_3H_5$ | |
| 466 | " | " | 3-CO-$N(CH_3)(cC_6H_{11})$ | |
| 467 | " | " | 3-COSH | |
| 468 | " | " | 3-COSNa | |
| 469 | " | " | 3-$COSCH_3$ | |
| 470 | " | " | 3-$COSC_2H_5$ | |
| 471 | " | " | 3-$COSCH_2C_6H_5$ | |
| 472 | " | " | 3-COS-n$C_8H_{17}$ | |
| 473 | " | " | 3-$COSC_2H_4OCH_3$ | |
| 474 | " | " | 3-$COSCH_2CHCH_2$ | |
| 475 | " | " | 3-$COSCH_2CCH$ | |
| 476 | " | " | 3-COS-c-$C_6H_{11}$ | |
| 477 | " | " | 3-$COSCH_2Si(CH_3)_3$ | |
| 478 | " | " | 3-COS-n-$C_4H_8CH(CH_3)_2$ | |
| 479 | " | " | 3-CON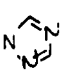 | |
| 480 | " | " | 3-$COOC_2H_4CH(CH_3)_2$ | |

Y=CH

| Beisp.-Nr. | $(R_1)_n$ | $R_2$ | X | Fp/$Kp_{Torr}$ [°C] |
|---|---|---|---|---|
| 481 | 2,4-$Cl_2$ | 5-c-$C_6H_{11}$ | 3-$COOCH_3$ | |
| 482 | " | " | 3-$COOC_2H_5$ | 106-108 |
| 483 | " | " | 3-COO-n-$C_3H_7$ | |
| 484 | " | " | 3-COO-i-$C_3H_7$ | |
| 485 | " | " | 3-COO-n-$C_4H_9$ | |
| 486 | " | " | 3-COO-n-$C_5H_{11}$ | |
| 487 | " | " | 3-COO-n-$C_6H_{13}$ | |
| 488 | " | " | 3-COO-n-$C_8H_{17}$ | |
| 489 | " | " | 3-COO-n-$C_{10}H_{21}$ | |
| 490 | " | " | 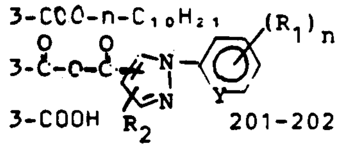 | |
| 491 | " | " | 3-COOH | 201-202 |
| 492 | " | " | 3-COOLi | |
| 493 | " | " | 3-COONa | |
| 494 | " | " | 3-COOK | |
| 495 | " | " | 3-$COOCa_{1/2}$ | |
| 496 | " | " | 3-COO-c-$C_4H_7$ | |
| 497 | " | " | 3-COO-c-$C_6H_{11}$ | |
| 498 | " | " | 3-$COOCH_2$-$C_6H_5$ | |
| 499 | " | " | 3-$COOCH_2$-(2,4-$Cl_2$-$C_6H_3$) | |
| 500 | " | " | 3-$COOCH_2CHCH_2$ | |
| 501 | " | " | 3-$COOC_2H_4CHCH_2$ | |
| 502 | " | " | 3-COO-n-$C_8H_{15}CHCH_2$ | |
| 503 | " | " | 3-COO-$CH_2CCH$ | |
| 504 | " | " | 3-COO-$C_2H_4$-CCH | |
| 505 | " | " | 3-COO-n-$C_5H_{10}CCH$ | |
| 506 | " | " | 3-$COOCH_2Si(CH_3)_3$ | |
| 507 | " | " | 3-$COOC_2H_4OCH_3$ | |
| 508 | " | " | 3-$CONH_2$ | |
| 509 | " | " | 3-CN | |
| 510 | " | " | 3-$CONHCH_3$ | |

Y=CH

| Beisp.-Nr. | $(R_1)_n$ | $R_2$ | X | $Fp/Kp_{Torr}$ [°C] |
|---|---|---|---|---|
| 511 | 2,4-$Cl_2$ | 5-c-$C_6H_{11}$ | 3-$CONHC_2H_5$ | 131-132 |
| 512 | " | " | 3-CONH-n-$C_3H_7$ | |
| 513 | " | " | 3-CONH-n-$C_4H_9$ | |
| 514 | " | " | 3-CONH-n-$C_6H_{13}$ | |
| 515 | " | " | 3-CONH-n-$C_{10}H_{21}$ | |
| 516 | " | " | 3-CONH-i-$C_3H_7$ | |
| 517 | " | " | 3-$CON(CH_3)_2$ | |
| 518 | " | " | 3-$CON(CH_3)(nC_6H_{13})$ | |
| 519 | " | " | 3-$CON(C_2H_5)_2$ | |
| 520 | " | " | 3-CO-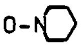 | |
| 521 | " | " | 3-CO-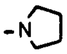 | |
| 522 | " | " | 3-CO-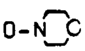 | |
| 523 | " | " | 3-CO-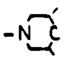 | |
| 524 | " | " | 3-CO-NH-c-$C_6H_{11}$ | |
| 525 | " | " | 3-CO-NH-c-$C_3H_5$ | |
| 526 | " | " | 3-CO-$N(CH_3)(cC_6H_{11})$ | |
| 527 | " | " | 3-COSH | |
| 528 | " | " | 3-COSNa | |
| 529 | " | " | 3-$COSCH_3$ | |
| 530 | " | " | 3-$COSC_2H_5$ | |
| 531 | " | " | 3-$COSCH_2C_6H_5$ | |
| 532 | " | " | 3-COS-n$C_8H_{17}$ | |
| 533 | " | " | 3-$COSC_2H_4OCH_3$ | |
| 534 | " | " | 3-$COSCH_2CHCH_2$ | |
| 535 | " | " | 3-$COSCH_2CCH$ | |
| 536 | " | " | 3-COS-c-$C_6H_{11}$ | |
| 537 | " | " | 3-$COSCH_2Si(CH_3)_3$ | |
| 538 | " | " | 3-COS-n-$C_4H_8CH(CH_3)_2$ | |
| 539 | " | " | 3-CO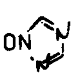 | |
| 540 | " | " | 3-$COOC_2H_4CH(CH_3)_2$ | |

Y=CH

| Beisp.-Nr. | $(R_1)_n$ | $R_2$ | X | $Fp/Kp_{Torr}$ [°C] |
|---|---|---|---|---|
| 541 | 2,4-$Br_2$ | 5-$CH_3$ | 3-$COOCH_3$ | |
| 542 | " | " | 3-$COOC_2H_5$ | 91-100 |
| 543 | " | " | 3-COO-n-$C_3H_7$ | |
| 544 | " | " | 3-COO-i-$C_3H_7$ | |
| 545 | " | " | 3-COO-n-$C_4H_9$ | |
| 546 | " | " | 3-COO-n-$C_5H_{11}$ | |
| 547 | " | " | 3-COO-n-$C_6H_{13}$ | |
| 548 | " | " | 3-COO-n-$C_8H_{17}$ | |
| 549 | " | " | 3-COO-n-$C_{10}H_{21}$ | |
| 550 | " | " | 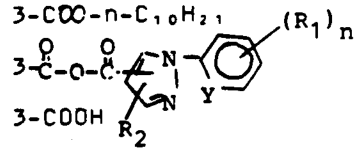 | |
| 551 | " | " | 3-COOH | |
| 552 | " | " | 3-COOLi | |
| 553 | " | " | 3-COONa | |
| 554 | " | " | 3-COOK | |
| 555 | " | " | 3-$COOCa_{1/2}$ | |
| 556 | " | " | 3-COO-c-$C_4H_7$ | |
| 557 | " | " | 3-COO-c-$C_6H_{11}$ | |
| 558 | " | " | 3-$COOCH_2$-$C_6H_5$ | |
| 559 | " | " | 3-$COOCH_2$-(2,4-$Cl_2$-$C_6H_3$) | |
| 560 | " | " | 3-$COOCH_2CHCH_2$ | |
| 561 | " | " | 3-$COOC_2H_4CHCH_2$ | |
| 562 | " | " | 3-COO-n-$C_8H_{16}CHCH_2$ | |
| 563 | " | " | 3-COO-$CH_2CCH$ | |
| 564 | " | " | 3-COO-$C_2H_4$-CCH | |
| 565 | " | " | 3-COO-n-$C_5H_{10}CCH$ | |
| 566 | " | " | 3-$COOCH_2Si(CH_3)_3$ | |
| 567 | " | " | 3-$COOC_2H_4OCH_3$ | |
| 568 | " | " | 3-$CONH_2$ | |
| 569 | " | " | 3-CN | |
| 570 | " | " | 3-$CONHCH_3$ | |

Y=CH

| Beisp.-Nr. | $(R_1)_n$ | $R_2$ | X | $Fp/Kp_{Torr}$ [°C] |
|---|---|---|---|---|
| 571 | 2,4-$Br_2$ | 5-$CH_3$ | 3-$CONHC_2H_5$ | |
| 572 | " | " | 3-CONH-n-$C_3H_7$ | |
| 573 | " | " | 3-CONH-n-$C_4H_9$ | |
| 574 | " | " | 3-CONH-n-$C_6H_{13}$ | |
| 575 | " | " | 3-CONH-n-$C_{10}H_{21}$ | |
| 576 | " | " | 3-CONH-i-$C_3H_7$ | |
| 577 | " | " | 3-$CON(CH_3)_2$ | |
| 578 | " | " | 3-$CON(CH_3)(nC_6H_{13})$ | |
| 579 | " | " | 3-$CON(C_2H_5)_2$ | |
| 580 | " | " | 3-CO-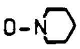 | |
| 581 | " | " | 3-CO-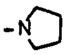 | |
| 582 | " | " | 3-CO-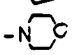 | |
| 583 | " | " | 3-CO-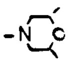 | |
| 584 | " | " | 3-CO-NH-c-$C_6H_{11}$ | |
| 585 | " | " | 3-CO-NH-c-$C_3H_5$ | |
| 586 | " | " | 3-CO-$N(CH_3)(cC_6H_{11})$ | |
| 587 | " | " | 3-COSH | |
| 588 | " | " | 3-COSNa | |
| 589 | " | " | 3-$COSCH_3$ | |
| 590 | " | " | 3-$COSC_2H_5$ | |
| 591 | " | " | 3-$COSCH_2C_6H_5$ | |
| 592 | " | " | 3-COS-n$C_8H_{17}$ | |
| 593 | " | " | 3-$COSC_2H_4OCH_3$ | |
| 594 | " | " | 3-$COSCH_2CHCH_2$ | |
| 595 | " | " | 3-$COSCH_2CCH$ | |
| 596 | " | " | 3-COS-c-$C_6H_{11}$ | |
| 597 | " | " | 3-$COSCH_2Si(CH_3)_3$ | |
| 598 | " | " | 3-COS-n-$C_4H_8CH(CH_3)_2$ | |
| 599 | " | " | 3-CON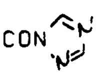 | |
| 600 | " | " | 3-$COOC_2H_4CH(CH_3)_2$ | |

| Y=CH Beisp.-Nr. | $(R_1)_n$ | $R_2$ | X | Fp/$Kp_{Torr}$ [°C] |
|---|---|---|---|---|
| 601 | $3-CF_3$ | $5-CH_3$ | $3-COOCH_3$ | |
| 602 | " | " | $3-COOC_2H_5$ | 73-75 |
| 603 | " | " | $3-COO-n-C_3H_7$ | |
| 604 | " | " | $3-COO-i-C_3H_7$ | |
| 605 | " | " | $3-COO-n-C_4H_9$ | Öl |
| 606 | " | " | $3-COO-n-C_5H_{11}$ | |
| 607 | " | " | $3-COO-n-C_6H_{13}$ | |
| 608 | " | " | $3-COO-n-C_8H_{17}$ | |
| 609 | " | " | $3-COO-n-C_{10}H_{21}$ | |
| 610 | " | " | 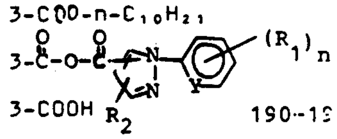 | |
| 611 | " | " | 3-COOH | 190-191 |
| 612 | " | " | 3-COOLi | |
| 613 | " | " | 3-COONa | |
| 614 | " | " | 3-COOK | |
| 615 | " | " | $3-COOCa_{1/2}$ | |
| 616 | " | " | $3-COO-c-C_4H_7$ | |
| 617 | " | " | $3-COO-c-C_6H_{11}$ | |
| 618 | " | " | $3-COOCH_2-C_6H_5$ | |
| 619 | " | " | $3-COOCH_2-(2,4-Cl_2-C_6H_3)$ | |
| 620 | " | " | $3-COOCH_2CHCH_2$ | |
| 621 | " | " | $3-COOC_2H_4CHCH_2$ | |
| 622 | " | " | $3-COO-n-C_8H_{16}CHCH_2$ | |
| 623 | " | " | $3-COO-CH_2CCH$ | |
| 624 | " | " | $3-COO-C_2H_4-CCH$ | |
| 625 | " | " | $3-COO-n-C_5H_{10}CCH$ | |
| 626 | " | " | $3-COOCH_2Si(CH_3)_3$ | |
| 627 | " | " | $3-COOC_2H_4OCH_3$ | |
| 628 | " | " | $3-CONH_2$ | |
| 629 | " | " | 3-CN | |
| 630 | " | " | $3-CONHCH_3$ | |

Y=CH

| Beisp.-Nr. | $(R_1)_n$ | $R_2$ | X | $Fp/Kp_{Torr}$ [°C] |
|---|---|---|---|---|
| 631 | $3-CF_3$ | $5-CH_3$ | $3-CONHC_2H_5$ | |
| 632 | " | " | $3-CONH-n-C_3H_7$ | 66-72 |
| 633 | " | " | $3-CONH-n-C_4H_9$ | |
| 634 | " | " | $3-CONH-n-C_6H_{13}$ | |
| 635 | " | " | $3-CONH-n-C_{10}H_{21}$ | |
| 636 | " | " | $3-CONH-i-C_3H_7$ | |
| 637 | " | " | $3-CON(CH_3)_2$ | |
| 638 | " | " | $3-CON(CH_3)(nC_6H_{13})$ | |
| 639 | " | " | $3-CON(C_2H_5)_2$ | |
| 640 | " | " | 3-CO-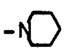 | |
| 641 | " | " | 3-CO-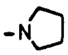 | |
| 642 | " | " | 3-CO-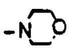 | |
| 643 | " | " | 3-CO-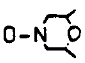 | |
| 644 | " | " | $3-CO-NH-c-C_6H_{11}$ | |
| 645 | " | " | $3-CO-NH-c-C_3H_5$ | |
| 646 | " | " | $3-CO-N(CH_3)(cC_6H_{11})$ | |
| 647 | " | " | 3-COSH | |
| 648 | " | " | 3-COSNa | |
| 649 | " | " | $3-COSCH_3$ | |
| 650 | " | " | $3-COSC_2H_5$ | |
| 651 | " | " | $3-COSCH_2C_6H_5$ | |
| 652 | " | " | $3-COS-nC_8H_{17}$ | |
| 653 | " | " | $3-COSC_2H_4OCH_3$ | |
| 654 | " | " | $3-COSCH_2CHCH_2$ | |
| 655 | " | " | $3-COSCH_2CCH$ | |
| 656 | " | " | $3-COS-c-C_6H_{11}$ | |
| 657 | " | " | $3-COSCH_2Si(CH_3)_3$ | |
| 658 | " | " | $3-COS-n-C_4H_8CH(CH_3)_2$ | |
| 659 | " | " | 3-CO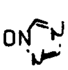 | |
| 660 | " | " | $3-COOC_2H_4CH(CH_3)_2$ | |

Y=CH

| Beisp.-Nr. | $(R_1)_n$ | $R_2$ | X | Fp/Kp$_{Torr}$ [°C] |
|---|---|---|---|---|
| 661 | 2,4-ClCF$_3$ | 5-CH$_3$ | 3-CONHC$_2$H$_5$ | |
| 662 | " | " | 3-CONH-n-C$_3$H$_7$ | 109-113 |
| 663 | " | " | 3-CONH-n-C$_4$H$_9$ | |
| 664 | " | " | 3-CONH-n-C$_6$H$_{13}$ | |
| 665 | " | " | 3-CONH-n-C$_{10}$H$_{21}$ | |
| 666 | " | " | 3-CONH-i-C$_3$H$_7$ | |
| 667 | " | " | 3-CON(CH$_3$)$_2$ | |
| 668 | " | " | 3-CON(CH$_3$)(nC$_6$H$_{13}$) | |
| 669 | " | " | 3-CON(C$_2$H$_5$)$_2$ | |
| 670 | " | " | 3-CO-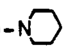 | |
| 671 | " | " | 3-CO-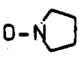 | |
| 672 | " | " | 3-CO-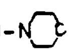 | |
| 673 | " | " | 3-CO-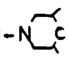 | |
| 674 | " | " | 3-CO-NH-c-C$_6$H$_{11}$ | |
| 675 | " | " | 3-CO-NH-c-C$_3$H$_5$ | |
| 676 | " | " | 3-CO-N(CH$_3$)(cC$_6$H$_{11}$) | |
| 677 | " | " | 3-COSH | |
| 678 | " | " | 3-COSNa | |
| 679 | " | " | 3-COSCH$_3$ | |
| 680 | " | " | 3-COSC$_2$H$_5$ | |
| 681 | " | " | 3-COSCH$_2$C$_6$H$_5$ | |
| 682 | " | " | 3-COS-nC$_8$H$_{17}$ | |
| 683 | " | " | 3-COSC$_2$H$_4$OCH$_3$ | |
| 684 | " | " | 3-COSCH$_2$CHCH$_2$ | |
| 685 | " | " | 3-COSCH$_2$CCH | |
| 686 | " | " | 3-COS-c-C$_6$H$_{11}$ | |
| 687 | " | " | 3-COSCH$_2$Si(CH$_3$)$_3$ | |
| 688 | " | " | 3-COS-n-C$_4$H$_8$CH(CH$_3$)$_2$ | |
| 689 | " | " | 3-CO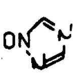 | |
| 690 | " | " | 3-COOC$_2$H$_4$CH(CH$_3$)$_2$ | |

Y=CH

| Beisp.-Nr. | $(R_1)_n$ | $R_2$ | X | $Fp/Kp_{Torr}$ [°C] |
|---|---|---|---|---|
| 691 | 2,4-ClCF$_3$ | 5-$CH_3$ | 3-$COOCH_3$ | |
| 692 | " | " | 3-$COOC_2H_5$ | |
| 693 | " | " | 3-COO-n-$C_3H_7$ | |
| 694 | " | " | 3-COO-i-$C_3H_7$ | |
| 695 | " | " | 3-COO-n-$C_4H_9$ | |
| 696 | " | " | 3-COO-n-$C_5H_{11}$ | |
| 697 | " | " | 3-COO-n-$C_6H_{13}$ | |
| 698 | " | " | 3-COO-n-$C_8H_{17}$ | |
| 699 | " | " | 3-COO-n-$C_{10}H_{21}$ | |
| 700 | " | " | 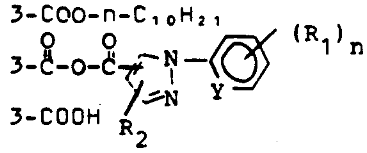 | |
| 701 | " | " | 3-COOH | |
| 702 | " | " | 3-COOLi | |
| 703 | " | " | 3-COONa | |
| 704 | " | " | 3-COOK | |
| 705 | " | " | 3-$COOCa_{1/2}$ | |
| 706 | " | " | 3-COO-c-$C_4H_7$ | |
| 707 | " | " | 3-COO-c-$C_6H_{11}$ | |
| 708 | " | " | 3-$COOCH_2$-$C_6H_5$ | |
| 709 | " | " | 3-$COOCH_2$-(2,4-$Cl_2$-$C_6H_3$) | |
| 710 | " | " | 3-$COOCH_2CHCH_2$ | |
| 711 | " | " | 3-$COOC_2H_4CHCH_2$ | |
| 712 | " | " | 3-COO-n-$C_8H_{16}CHCH_2$ | |
| 713 | " | " | 3-COO-$CH_2CCH$ | |
| 714 | " | " | 3-COO-$C_2H_4$-CCH | |
| 715 | " | " | 3-COO-n-$C_5H_{10}CCH$ | |
| 716 | " | " | 3-$COOCH_2Si(CH_3)_3$ | |
| 717 | " | " | 3-$COOC_2H_4OCH_3$ | |
| 718 | " | " | 3-$CONH_2$ | |
| 719 | " | " | 3-CN | |
| 720 | " | " | 3-$CONHCH_3$ | |

Y=CH

| Beisp.-Nr. | $(R_1)_n$ | $R_2$ | X | Fp/$Kp_{Torr}$ [°C] |
|---|---|---|---|---|
| 721 | 4,2-ClCF$_3$ | 5-CH$_3$ | 3-COOCH$_3$ | |
| 722 | " | " | 3-COOC$_2$H$_5$ | 49-51 |
| 723 | " | " | 3-COO-n-C$_3$H$_7$ | |
| 724 | " | " | 3-COO-i-C$_3$H$_7$ | |
| 725 | " | " | 3-COO-n-C$_4$H$_9$ | |
| 726 | " | " | 3-COO-n-C$_5$H$_{11}$ | |
| 727 | " | " | 3-COO-n-C$_6$H$_{13}$ | |
| 728 | " | " | 3-COO-n-C$_8$H$_{17}$ | |
| 729 | " | " | 3-COO-n-C$_{10}$H$_{21}$ | |
| 730 | " | " | 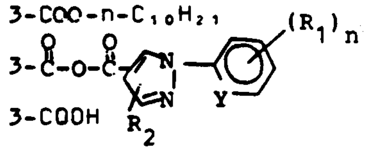 | |
| 731 | " | " | 3-COOH | |
| 732 | " | " | 3-COOLi | |
| 733 | " | " | 3-COONa | |
| 734 | " | " | 3-COOK | |
| 735 | " | " | 3-COOCa$_{1/2}$ | |
| 736 | " | " | 3-COO-c-C$_4$H$_7$ | |
| 737 | " | " | 3-COO-c-C$_6$H$_{11}$ | |
| 738 | " | " | 3-COOCH$_2$-C$_6$H$_5$ | |
| 739 | " | " | 3-COOCH$_2$-(2,4-Cl$_2$-C$_6$H$_3$) | |
| 740 | " | " | 3-COOCH$_2$CHCH$_2$ | |
| 741 | " | " | 3-COOC$_2$H$_4$CHCH$_2$ | |
| 742 | " | " | 3-COO-n-C$_8$H$_{16}$CHCH$_2$ | |
| 743 | " | " | 3-COO-CH$_2$CCH | |
| 744 | " | " | 3-COO-C$_2$H$_4$-CCH | |
| 745 | " | " | 3-COO-n-C$_5$H$_{10}$CCH | |
| 746 | " | " | 3-COOCH$_2$Si(CH$_3$)$_3$ | |
| 747 | " | " | 3-COOC$_2$H$_4$OCH$_3$ | |
| 748 | " | " | 3-CONH$_2$ | |
| 749 | " | " | 3-CN | |
| 750 | " | " | 3-CONHCH$_3$ | |

Y=CH

| Beisp.-Nr. | $(R_1)_n$ | $R_2$ | X | Fp/$Kp_{Torr}$ [°C] |
|---|---|---|---|---|
| 751 | 4,2-$ClCF_3$ | 5-$CH_3$ | 3-$CONHC_2H_5$ | |
| 752 | " | " | 3-CONH-n-$C_3H_7$ | |
| 753 | " | " | 3-CONH-n-$C_4H_9$ | |
| 754 | " | " | 3-CONH-n-$C_6H_{13}$ | |
| 755 | " | " | 3-CONH-n-$C_{10}H_{21}$ | |
| 756 | " | " | 3-CONH-i-$C_3H_7$ | |
| 757 | " | " | 3-$CON(CH_3)_2$ | |
| 758 | " | " | 3-$CON(CH_3)(nC_6H_{13})$ | |
| 759 | " | " | 3-$CON(C_2H_5)_2$ | |
| 760 | " | " | 3-CO-N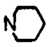 | |
| 761 | " | " | 3-CO-N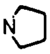 | |
| 762 | " | " | 3-CO-N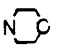 | |
| 763 | " | " | 3-CO-N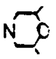 | |
| 764 | " | " | 3-CO-NH-c-$C_6H_{11}$ | |
| 765 | " | " | 3-CO-NH-c-$C_3H_5$ | |
| 766 | " | " | 3-CO-$N(CH_3)(cC_6H_{11})$ | |
| 767 | " | " | 3-COSH | |
| 768 | " | " | 3-COSNa | |
| 769 | " | " | 3-$COSCH_3$ | |
| 770 | " | " | 3-$COSC_2H_5$ | |
| 771 | " | " | 3-$COSCH_2C_6H_5$ | |
| 772 | " | " | 3-COS-n$C_8H_{17}$ | |
| 773 | " | " | 3-$COSC_2H_4OCH_3$ | |
| 774 | " | " | 3-$COSCH_2CHCH_2$ | |
| 775 | " | " | 3-$COSCH_2CCH$ | |
| 776 | " | " | 3-COS-c-$C_6H_{11}$ | |
| 777 | " | " | 3-$COSCH_2Si(CH_3)_3$ | |
| 778 | " | " | 3-COS-n-$C_4H_8CH(CH_3)_2$ | |
| 779 | " | " | 3-CON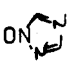 | |
| 780 | " | " | 3-$COOC_2H_4CH(CH_3)_2$ | |

| Y=CH Beisp.-Nr. | $(R_1)_n$ | $R_2$ | X | $Fp/Kp_{Torr}$ [°C] |
|---|---|---|---|---|
| 781 | 2,6,4-$Cl_2CF_3$ | 5-$CH_3$ | 3-$COOCH_3$ | |
| 782 | " | " | 3-$COOC_2H_5$ | 138-140 |
| 783 | " | " | 3-COO-n-$C_3H_7$ | |
| 784 | " | " | 3-COO-i-$C_3H_7$ | |
| 785 | " | " | 3-COO-n-$C_4H_9$ | |
| 786 | " | " | 3-COO-n-$C_5H_{11}$ | |
| 787 | " | " | 3-COO-n-$C_6H_{13}$ | |
| 788 | " | " | 3-COO-n-$C_3H_{17}$ | |
| 789 | " | " | 3-COO-n-$C_{10}H_{21}$ | |
| 790 | " | " | 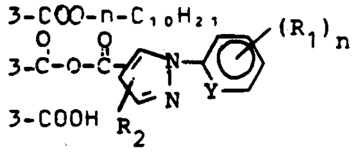 | |
| 791 | " | " | 3-COOH | |
| 792 | " | " | 3-COOLi | |
| 793 | " | " | 3-COONa | |
| 794 | " | " | 3-COOK | |
| 795 | " | " | 3-$COOCa_{1/2}$ | |
| 796 | " | " | 3-COO-c-$C_4H_7$ | |
| 797 | " | " | 3-COO-c-$C_6H_{11}$ | |
| 798 | " | " | 3-$COOCH_2$-$C_6H_5$ | |
| 799 | " | " | 3-$COOCH_2$-(2,4-$Cl_2$-$C_6H_3$) | |
| 800 | " | " | 3-$COOCH_2CHCH_2$ | |
| 801 | " | " | 3-$COOC_2H_4CHCH_2$ | |
| 802 | " | " | 3-COO-n-$C_8H_{16}CHCH_2$ | |
| 803 | " | " | 3-COO-$CH_2CCH$ | |
| 804 | " | " | 3-COO-$C_2H_4$-CCH | |
| 805 | " | " | 3-COO-n-$C_5H_{10}CCH$ | |
| 806 | " | " | 3-$COOCH_2Si(CH_3)_3$ | |
| 807 | " | " | 3-$COOC_2H_4OCH_3$ | |
| 808 | " | " | 3-$CONH_2$ | |
| 809 | " | " | 3-CN | |
| 810 | " | " | 3-$CONHCH_3$ | |

Y=CH

| Beisp.-Nr. | $(R_1)_n$ | $R_2$ | X | Fp/$Kp_{Torr}$ [°C] |
|---|---|---|---|---|
| 811 | 2,6,4-$Cl_2CF_3$ | 5-$CH_3$ | 3-$CONHC_2H_5$ | |
| 812 | " | " | 3-CONH-n-$C_3H_7$ | |
| 813 | " | " | 3-CONH-n-$C_4H_9$ | |
| 814 | " | " | 3-CONH-n-$C_6H_{13}$ | |
| 815 | " | " | 3-CONH-n-$C_{10}H_{21}$ | |
| 816 | " | " | 3-CONH-i-$C_3H_7$ | |
| 817 | " | " | 3-$CON(CH_3)_2$ | |
| 818 | " | " | 3-$CON(CH_3)(nC_6H_{13})$ | |
| 819 | " | " | 3-$CON(C_2H_5)_2$ | |
| 820 | " | " | 3-CO-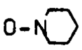 | |
| 821 | " | " | 3-CO-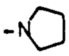 | |
| 822 | " | " | 3-CO-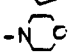 | |
| 823 | " | " | 3-CO-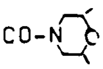 | |
| 824 | " | " | 3-CO-NH-c-$C_6H_{11}$ | |
| 825 | " | " | 3-CO-NH-c-$C_3H_5$ | |
| 826 | " | " | 3-CO-$N(CH_3)(cC_6H_{11})$ | |
| 827 | " | " | 3-COSH | |
| 828 | " | " | 3-COSNa | |
| 829 | " | " | 3-$COSCH_3$ | |
| 830 | " | " | 3-$COSC_2H_5$ | |
| 831 | " | " | 3-$COSCH_2C_6H_5$ | |
| 832 | " | " | 3-COS-n$C_8H_{17}$ | |
| 833 | " | " | 3-$COSC_2H_4OCH_3$ | |
| 834 | " | " | 3-$COSCH_2CHCH_2$ | |
| 835 | " | " | 3-$COSCH_2CCH$ | |
| 836 | " | " | 3-COS-c-$C_6H_{11}$ | |
| 837 | " | " | 3-$COSCH_2Si(CH_3)_3$ | |
| 838 | " | " | 3-COS-n-$C_4H_8CH(CH_3)_2$ | |
| 839 | " | " | 3-CO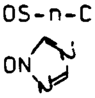 | |
| 840 | " | " | 3-$COOC_2H_4CH(CH_3)_2$ | |

Y=N

| Beisp.-Nr. | $(R_1)_n$ | $R_2$ | X | Fp/$Kp_{Torr}$ [°C] |
|---|---|---|---|---|
| 841 | 3,5-Cl -$CF_3$ | 5-$CH_3$ | 3-$COOCH_3$ | |
| 842 | " | " | 3-$COOC_2H_5$ | 55-53 |
| 843 | " | " | 3-COO-n-$C_3H_7$ | |
| 844 | " | " | 3-COO-i-$C_3H_7$ | |
| 845 | " | " | 3-COO-n-$C_4H_9$ | |
| 846 | " | " | 3-COO-n-$C_5H_{11}$ | |
| 847 | " | " | 3-COO-n-$C_6H_{13}$ | |
| 848 | " | " | 3-COO-n-$C_8H_{17}$ | |
| 849 | " | " | 3-COO-n-$C_{10}H_{21}$ | |
| 850 | " | " | 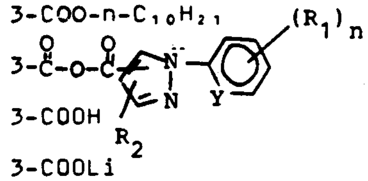 | |
| 851 | " | " | 3-COOH | |
| 852 | " | " | 3-COOLi | |
| 853 | " | " | 3-COONa | |
| 854 | " | " | 3-COOK | |
| 855 | " | " | 3-$COOCa_{1/2}$ | |
| 856 | " | " | 3-COO-c-$C_4H_7$ | |
| 857 | " | " | 3-COO-c-$C_6H_{11}$ | |
| 858 | " | " | 3-$COOCH_2$-$C_6H_5$ | |
| 859 | " | " | 3-$COOCH_2$-(2,4-$Cl_2$-$C_6H_3$) | |
| 860 | " | " | 3-$COOCH_2CHCH_2$ | |
| 861 | " | " | 3-$COOC_2H_4CHCH_2$ | |
| 862 | " | " | 3-COO-n-$C_8H_{15}CHCH_2$ | |
| 863 | " | " | 3-COO-$CH_2CCH$ | |
| 864 | " | " | 3-COO-$C_2H_4$-CCH | |
| 865 | " | " | 3-COO-n-$C_5H_{10}CCH$ | |
| 866 | " | " | 3-$COOCH_2Si(CH_3)_3$ | |
| 867 | " | " | 3-$COOC_2H_4OCH_3$ | |
| 868 | " | " | 3-$CONH_2$ | |
| 869 | " | " | 3-CN | |
| 870 | " | " | 3-$CONHCH_3$ | |

Y=N

| Beisp.-Nr. | $(R_1)_n$ | $R_2$ | X | $Fp/Kp_{Torr}$ [°C] |
|---|---|---|---|---|
| 871 | 3,5-ClCF$_3$ | 5-$CH_3$ | 3-$CONHC_2H_5$ | |
| 872 | " | " | 3-CONH-n-$C_3H_7$ | |
| 873 | " | " | 3-CONH-n-$C_4H_9$ | |
| 874 | " | " | 3-CONH-n-$C_6H_{13}$ | |
| 875 | " | " | 3-CONH-n-$C_{10}H_{21}$ | |
| 876 | " | " | 3-CONH-i-$C_3H_7$ | |
| 877 | " | " | 3-$CON(CH_3)_2$ | |
| 878 | " | " | 3-$CON(CH_3)(nC_6H_{13})$ | |
| 879 | " | " | 3-$CON(C_2H_5)_2$ | |
| 880 | " | " | 3-CO-N 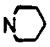 | |
| 881 | " | " | 3-CO-N 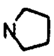 | |
| 882 | " | " | 3-CO-N 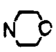 | |
| 883 | " | " | 3-CO-N 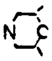 | |
| 884 | " | " | 3-CO-NH-c-$C_6H_{11}$ | |
| 885 | " | " | 3-CO-NH-c-$C_3H_5$ | |
| 886 | " | " | 3-CO-N$(CH_3)(cC_6H_{11})$ | |
| 887 | " | " | 3-COSH | |
| 888 | " | " | 3-COSNa | |
| 889 | " | " | 3-$COSCH_3$ | |
| 890 | " | " | 3-$COSC_2H_5$ | |
| 891 | " | " | 3-$COSCH_2C_6H_5$ | |
| 892 | " | " | 3-COS-n$C_8H_{17}$ | |
| 893 | " | " | 3-$COSC_2H_4OCH_3$ | |
| 894 | " | " | 3-$COSCH_2CHCH_2$ | |
| 895 | " | " | 3-$COSCH_2CCH$ | |
| 896 | " | " | 3-COS-c-$C_6H_{11}$ | |
| 897 | " | " | 3-$COSCH_2Si(CH_3)_3$ | |
| 898 | " | " | 3-COS-n-$C_4H_8CH(CH_3)_2$ | |
| 899 | " | " | 3-CON 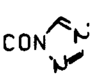 | |
| 900 | " | " | 3-$COOC_2H_4CH(CH_3)_2$ | |

| Y=N Beisp.-Nr. | $(R_1)_n$ | $R_2$ | X | $Fp/Kp_{Torr}$ [°C] |
|---|---|---|---|---|
| 901 | 3,5-ClCF$_3$ | 3-CH$_3$ | 5-COOCH$_3$ | |
| 902 | " | " | 5-COOC$_2$H$_5$ | 81 |
| 903 | " | " | 5-COO-n-C$_3$H$_7$ | |
| 904 | " | " | 5-COO-i-C$_3$H$_7$ | |
| 905 | " | " | 5-COO-n-C$_4$H$_9$ | |
| 906 | " | " | 5-COO-n-C$_5$H$_{11}$ | |
| 907 | " | " | 5-COO-n-C$_6$H$_{13}$ | |
| 908 | " | " | 5-COO-n-C$_8$H$_{17}$ | |
| 909 | " | " | 5-COO-n-C$_{10}$H$_{21}$ | |
| 910 | " | " | 5-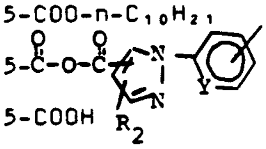 | |
| 911 | " | " | 5-COOH | |
| 912 | " | " | 5-COOLi | |
| 913 | " | " | 5-COONa | |
| 914 | " | " | 5-COOK | |
| 915 | " | " | 5-COOCa$_{1/2}$ | |
| 916 | " | " | 5-COO-c-C$_4$H$_7$ | |
| 917 | " | " | 5-COO-c-C$_6$H$_{11}$ | |
| 918 | " | " | 5-COOCH$_2$-C$_6$H$_5$ | |
| 919 | " | " | 5-COOCH$_2$-(2,4-Cl$_2$-C$_6$H$_3$) | |
| 920 | " | " | 5-COOCH$_2$CHCH$_2$ | |
| 921 | " | " | 5-COOC$_2$H$_4$CHCH$_2$ | |
| 922 | " | " | 5-COO-n-C$_8$H$_{15}$CHCH$_2$ | |
| 923 | " | " | 5-COO-CH$_2$CCH | |
| 924 | " | " | 5-COO-C$_2$H$_4$-CCH | |
| 925 | " | " | 5-COO-n-C$_5$H$_{10}$CCH | |
| 926 | " | " | 5-COOCH$_2$Si(CH$_3$)$_3$ | |
| 927 | " | " | 5-COOC$_2$H$_4$OCH$_3$ | |
| 928 | " | " | 5-CONH$_2$ | |
| 929 | " | " | 5-CN | |
| 930 | " | " | 5-CONHCH$_3$ | |

Y=N

| Beisp.-Nr. | $(R_1)_n$ | $R_2$ | X | Fp/$Kp_{Torr}$ [°C] |
|---|---|---|---|---|
| 931 | 3,5-Cl-$CF_3$ | 3-$CH_3$ | 5-$CONHC_2H_5$ | |
| 932 | " | " | 5-CONH-n-$C_3H_7$ | |
| 933 | " | " | 5-CONH-n-$C_4H_9$ | |
| 934 | " | " | 5-CONH-n-$C_6H_{13}$ | |
| 935 | " | " | 5-CONH-n-$C_{10}H_{21}$ | |
| 936 | " | " | 5-CONH-i-$C_3H_7$ | |
| 937 | " | " | 5-$CON(CH_3)_2$ | |
| 938 | " | " | 5-$CON(CH_3)(nC_6H_{13})$ | |
| 939 | " | " | 5-$CON(C_2H_5)_2$ | |
| 940 | " | " | 5-CO-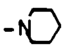 | |
| 941 | " | " | 5-CO-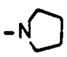 | |
| 942 | " | " | 5-CO-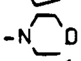 | |
| 943 | " | " | 5-CO-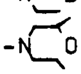 | |
| 944 | " | " | 5-CO-NH-c-$C_6H_{11}$ | |
| 945 | " | " | 5-CO-NH-c-$C_3H_5$ | |
| 946 | " | " | 5-CO-$N(CH_3)(cC_6H_{11})$ | |
| 947 | " | " | 5-COSH | |
| 948 | " | " | 5-COSNa | |
| 949 | " | " | 5-$COSCH_3$ | |
| 950 | " | " | 5-$COSC_2H_5$ | |
| 951 | " | " | 5-$COSCH_2C_6H_5$ | |
| 952 | " | " | 5-COS-n$C_8H_{17}$ | |
| 953 | " | " | 5-$COSC_2H_4OCH_3$ | |
| 954 | " | " | 5-$COSCH_2CHCH_2$ | |
| 955 | " | " | 5-$COSCH_2CCH$ | |
| 956 | " | " | 5-COS-c-$C_6H_{11}$ | |
| 957 | " | " | 5-$COSCH_2Si(CH_3)_3$ | |
| 958 | " | " | 5-COS-n-$C_4H_8CH(CH_3)_2$ | |
| 959 | " | " | 5-CO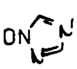 | |
| 960 | " | " | 5-$COOC_2H_4CH(CH_3)_2$ | |

Y=CH

| Beisp.-Nr. | $(R_1)_n$ | $R_2$ | X | Fp/$Kp_{Torr}$ [°C] |
|---|---|---|---|---|
| 961 | 2,3-$Cl_2$ | 5-$CH_3$ | 3-$COOCH_3$ | |
| 962 | " | " | 3-$COOC_2H_5$ | 77-79 |
| 963 | " | " | 3-COO-n-$C_3H_7$ | |
| 964 | " | " | 3-COO-i-$C_3H_7$ | |
| 965 | " | " | 3-COO-n-$C_4H_9$ | |
| 966 | " | " | 3-COO-n-$C_5H_{11}$ | |
| 967 | " | " | 3-COO-n-$C_6H_{13}$ | |
| 968 | " | " | 3-COO-n-$C_8H_{17}$ | |
| 969 | " | " | 3-COO-n-$C_{10}H_{21}$ | |
| 970 | " | " | 3- 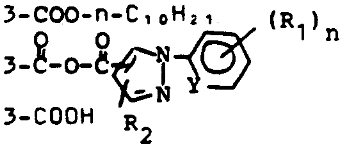 | |
| 971 | " | " | 3-COOH | |
| 972 | " | " | 3-COOLi | |
| 973 | " | " | 3-COONa | |
| 974 | " | " | 3-COOK | |
| 975 | " | " | 3-$COOCa_{1/2}$ | |
| 976 | " | " | 3-COO-c-$C_4H_7$ | |
| 977 | " | " | 3-COO-c-$C_6H_{11}$ | |
| 978 | " | " | 3-$COOCH_2$-$C_6H_5$ | |
| 979 | " | " | 3-$COOCH_2$-(2,4-$Cl_2$-$C_6H_3$) | |
| 980 | " | " | 3-$COOCH_2CHCH_2$ | |
| 981 | " | " | 3-$COOC_2H_4CHCH_2$ | |
| 982 | " | " | 3-COO-n-$C_8H_{16}CHCH_2$ | |
| 983 | " | " | 3-COO-$CH_2CCH$ | |
| 984 | " | " | 3-COO-$C_2H_4$-CCH | |
| 985 | " | " | 3-COO-n-$C_5H_{10}CCH$ | |
| 986 | " | " | 3-$COOCH_2Si(CH_3)_3$ | |
| 987 | " | " | 3-$COOC_2H_4OCH_3$ | |
| 988 | " | " | 3-$CONH_2$ | |
| 989 | " | " | 3-CN | |
| 990 | " | " | 3-$CONHCH_3$ | |

Y=CH

| Beisp.-Nr. | $(R_1)_n$ | $R_2$ | X | $Fp/Kp_{Torr}$ [°C] |
|---|---|---|---|---|
| 991 | 2,3-$Cl_2$ | 5-$CH_3$ | 3-$CONHC_2H_5$ | |
| 992 | " | " | 3-CONH-n-$C_3H_7$ | |
| 993 | " | " | 3-CONH-n-$C_4H_9$ | |
| 994 | " | " | 3-CONH-n-$C_6H_{13}$ | |
| 995 | " | " | 3-CONH-n-$C_{10}H_{21}$ | |
| 996 | " | " | 3-CONH-i-$C_3H_7$ | |
| 997 | " | " | 3-$CON(CH_3)_2$ | |
| 998 | " | " | 3-$CON(CH_3)(nC_6H_{13})$ | |
| 999 | " | " | 3-$CON(C_2H_5)_2$ | |
| 1000 | " | " | 3-CO-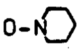 | |
| 1001 | " | " | 3-CO-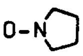 | |
| 1002 | " | " | 3-CO-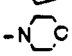 | |
| 1003 | " | " | 3-CO-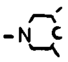 | |
| 1004 | " | " | 3-CO-NH-c-$C_6H_{11}$ | |
| 1005 | " | " | 3-CO-NH-c-$C_3H_5$ | |
| 1006 | " | " | 3-CO-$N(CH_3)(cC_6H_{11})$ | |
| 1007 | " | " | 3-COSH | |
| 1008 | " | " | 3-COSNa | |
| 1009 | " | " | 3-$COSCH_3$ | |
| 1010 | " | " | 3-$COSC_2H_5$ | |
| 1011 | " | " | 3-$COSCH_2C_6H_5$ | |
| 1012 | " | " | 3-COS-$nC_8H_{17}$ | |
| 1013 | " | " | 3-$COSC_2H_4OCH_3$ | |
| 1014 | " | " | 3-$COSCH_2CHCH_2$ | |
| 1015 | " | " | 3-$COSCH_2CCH$ | |
| 1016 | " | " | 3-COS-c-$C_6H_{11}$ | |
| 1017 | " | " | 3-$COSCH_2Si(CH_3)_3$ | |
| 1018 | " | " | 3-COS-n-$C_4H_8CH(CH_3)_2$ | |
| 1019 | " | " | 3-CO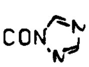 | |
| 1020 | " | " | 3-$COOC_2H_4CH(CH_3)_2$ | |

Y=CH

| Beisp.-Nr. | $(R_1)_n$ | $R_2$ | X | Fp/$Kp_{Torr}$ [°C] |
|---|---|---|---|---|
| 1021 | 2,4,5-$Cl_3$;$OCH_3$ | 5-$CH_3$ | 3-$COOCH_3$ | |
| 1022 | " | " | 3-$COOC_2H_5$ | 155-159 |
| 1023 | " | " | 3-COO-n-$C_3H_7$ | |
| 1024 | " | " | 3-COO-i-$C_3H_7$ | |
| 1025 | " | " | 3-COO-n-$C_4H_9$ | |
| 1026 | " | " | 3-COO-n-$C_5H_{11}$ | |
| 1027 | " | " | 3-COO-n-$C_6H_{13}$ | |
| 1028 | " | " | 3-COO-n-$C_8H_{17}$ | |
| 1029 | " | " | 3-COO-n-$C_{10}H_{21}$ | |
| 1030 | " | " | 3- 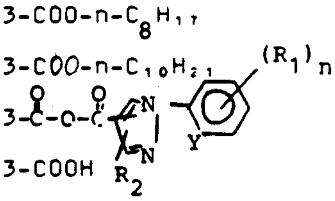 | |
| 1031 | " | " | 3-COOH | |
| 1032 | " | " | 3-COOLi | |
| 1033 | " | " | 3-COONa | |
| 1034 | " | " | 3-COOK | |
| 1035 | " | " | 3-$COOCa_{1/2}$ | |
| 1036 | " | " | 3-COO-c-$C_4H_7$ | |
| 1037 | " | " | 3-COO-c-$C_6H_{11}$ | |
| 1038 | " | " | 3-$COOCH_2$-$C_6H_5$ | |
| 1039 | " | " | 3-$COOCH_2$-(2,4-$Cl_2$-$C_6H_3$) | |
| 1040 | " | " | 3-$COOCH_2CHCH_2$ | |
| 1041 | " | " | 3-$COOC_2H_4CHCH_2$ | |
| 1042 | " | " | 3-COO-n-$C_8H_{16}CHCH_2$ | |
| 1043 | " | " | 3-COO-$CH_2CCH$ | |
| 1044 | " | " | 3-COO-$C_2H_4$-CCH | |
| 1045 | " | " | 3-COO-n-$C_5H_{10}CCH$ | |
| 1046 | " | " | 3-$COOCH_2Si(CH_3)_3$ | |
| 1047 | " | " | 3-$COOC_2H_4OCH_3$ | |
| 1048 | " | " | 3-$CONH_2$ | |
| 1049 | " | " | 3-CN | |
| 1050 | " | " | 3-$CONHCH_3$ | |

Y=CH

| Beisp.-Nr. | $(R_1)_n$ | $R_2$ | X | Fp/$Kp_{Torr}$ [°C] |
|---|---|---|---|---|
| 1051 | 2,4,5-$Cl_2OCH_3$ | 5-$CH_3$ | 3-$CONHC_2H_5$ | |
| 1052 | " | " | 3-CONH-n-$C_3H_7$ | |
| 1053 | " | " | 3-CONH-n-$C_4H_9$ | |
| 1054 | " | " | 3-CONH-n-$C_6H_{13}$ | |
| 1055 | " | " | 3-CONH-n-$C_{10}H_{21}$ | |
| 1056 | " | " | 3-CONH-i-$C_3H_7$ | |
| 1057 | " | " | 3-$CON(CH_3)_2$ | |
| 1058 | " | " | 3-$CON(CH_3)(nC_6H_{13})$ | |
| 1059 | " | " | 3-$CON(C_2H_5)_2$ | |
| 1060 | " | " | 3-CO-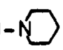 | |
| 1061 | " | " | 3-CO-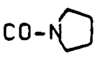 | |
| 1062 | " | " | 3-CO-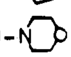 | |
| 1063 | " | " | 3-CO-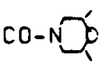 | |
| 1064 | " | " | 3-CO-NH-c-$C_6H_{11}$ | |
| 1065 | " | " | 3-CO-NH-c-$C_3H_5$ | |
| 1066 | " | " | 3-CO-$N(CH_3)(cC_6H_{11})$ | |
| 1067 | " | " | 3-COSH | |
| 1068 | " | " | 3-COSNa | |
| 1069 | " | " | 3-$COSCH_3$ | |
| 1070 | " | " | 3-$COSC_2H_5$ | |
| 1071 | " | " | 3-$COSCH_2C_6H_5$ | |
| 1072 | " | " | 3-COS-$nC_8H_{17}$ | |
| 1073 | " | " | 3-$COSC_2H_4OCH_3$ | |
| 1074 | " | " | 3-$COSCH_2CHCH_2$ | |
| 1075 | " | " | 3-$COSCH_2CCH$ | |
| 1076 | " | " | 3-COS-c-$C_6H_{11}$ | |
| 1077 | " | " | 3-$COSCH_2Si(CH_3)_3$ | |
| 1078 | " | " | 3-COS-n-$C_4H_8CH(CH_3)_2$ | |
| 1079 | " | " | 3-CO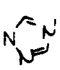 | |
| 1080 | " | " | 3-$COOC_2H_4CH(CH_3)_2$ | |

Y=CH

| Beisp.-Nr. | $(R_1)_n$ | $R_2$ | X | $Fp/Kp_{Torr}$ [°C] |
|---|---|---|---|---|
| 1081 | 2,4,5-$Cl_2OCH_3$ | 3-$CH_3$ | 5-$COOCH_3$ | |
| 1082 | " | " | 5-$COOC_2H_5$ | Öl |
| 1083 | " | " | 5-COO-n-$C_3H_7$ | |
| 1084 | " | " | 5-COO-i-$C_3H_7$ | |
| 1085 | " | " | 5-COO-n-$C_4H_9$ | |
| 1086 | " | " | 5-COO-n-$C_5H_{11}$ | |
| 1087 | " | " | 5-COO-n-$C_6H_{13}$ | |
| 1088 | " | " | 5-COO-n-$C_8H_{17}$ | |
| 1089 | " | " | 5-COO-n-$C_{10}H_{21}$ | |
| 1090 | " | " | 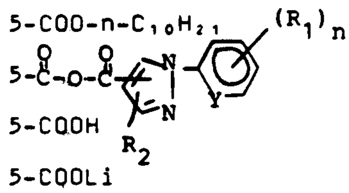 | |
| 1091 | " | " | 5-COOH | |
| 1092 | " | " | 5-COOLi | |
| 1093 | " | " | 5-COONa | |
| 1094 | " | " | 5-COOK | |
| 1095 | " | " | 5-$COOCa_{1/2}$ | |
| 1096 | " | " | 5-COO-c-$C_4H_7$ | |
| 1097 | " | " | 5-COO-c-$C_6H_{11}$ | |
| 1098 | " | " | 5-$COOCH_2$-$C_6H_5$ | |
| 1099 | " | " | 5-$COOCH_2$-(2,4-$Cl_2$-$C_6H_3$) | |
| 1100 | " | " | 5-$COOCH_2CHCH_2$ | |
| 1101 | " | " | 5-$COOC_2H_4CHCH_2$ | |
| 1102 | " | " | 5-COO-n-$C_8H_{16}$ $CHCH_2$ | |
| 1103 | " | " | 5-COO-$CH_2CCH$ | |
| 1104 | " | " | 5-COO-$C_2H_4$-CCH | |
| 1105 | " | " | 5-COO-n-$C_5H_{10}CCH$ | |
| 1106 | " | " | 5-$COOCH_2Si(CH_3)_3$ | |
| 1107 | " | " | 5-$COOC_2H_4OCH_3$ | |
| 1108 | " | " | 5-$CONH_2$ | |
| 1109 | " | " | 5-CN | |
| 1110 | " | " | 5-$CONHCH_3$ | |

Y=CH

| Beisp.-Nr. | $(R_1)_n$ | $R_2$ | X | Fp/$Kp_{Torr}$ [°C] |
|---|---|---|---|---|
| 1111 | 2,4,5-$Cl_2OCH_3$ | 3-$CH_3$ | 5-$CONHC_2H_5$ | |
| 1112 | " | " | 5-CONH-n-$C_3H_7$ | |
| 1113 | " | " | 5-CONH-n-$C_4H_9$ | |
| 1114 | " | " | 5-CONH-n-$C_6H_{13}$ | |
| 1115 | " | " | 5-CONH-n-$C_{10}H_{21}$ | |
| 1116 | " | " | 5-CONH-i-$C_3H_7$ | |
| 1117 | " | " | 5-$CON(CH_3)_2$ | |
| 1118 | " | " | 5-$CON(CH_3)(nC_6H_{13})$ | |
| 1119 | " | " | 5-$CON(C_2H_5)_2$ | |
| 1120 | " | " | 5-CO-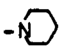 | |
| 1121 | " | " | 5-CO-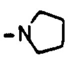 | |
| 1122 | " | " | 5-CO-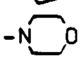 | |
| 1123 | " | " | 5-CO-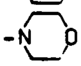 | |
| 1124 | " | " | 5-CO-NH-c-$C_6H_{11}$ | |
| 1125 | " | " | 5-CO-NH-c-$C_3H_5$ | |
| 1126 | " | " | 5-CO-$N(CH_3)(cC_6H_{11})$ | |
| 1127 | " | " | 5-COSH | |
| 1128 | " | " | 5-COSNa | |
| 1129 | " | " | 5-$COSCH_3$ | |
| 1130 | " | " | 5-$COSC_2H_5$ | |
| 1131 | " | " | 5-$COSCH_2C_6H_5$ | |
| 1132 | " | " | 5-COS-$nC_8H_{17}$ | |
| 1133 | " | " | 5-$COSC_2H_4OCH_3$ | |
| 1134 | " | " | 5-$COSCH_2CHCH_2$ | |
| 1135 | " | " | 5-$COSCH_2CCH$ | |
| 1136 | " | " | 5-COS-c-$C_6H_{11}$ | |
| 1137 | " | " | 5-$COSCH_2Si(CH_3)_3$ | |
| 1138 | " | " | 5-COS-n-$C_4H_8CH(CH_3)_2$ | |
| 1139 | " | " | 5-CON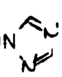 | |
| 1140 | " | " | 5-$COOC_2H_4CH(CH_3)_2$ | |

Y=CH

| Beisp.-Nr. | $(R_1)_n$ | $R_2$ | X | $Fp/Kp_{Torr}$ [°C] |
|---|---|---|---|---|
| 1141 | $2,6,3-(C_2H_5)_2Cl$ | $5-CH_3$ | $3-COOCH_3$ | |
| 1142 | " | " | $3-COOC_2H_5$ | Öl |
| 1143 | " | " | $3-COO-n-C_3H_7$ | |
| 1144 | " | " | $3-COO-i-C_3H_7$ | |
| 1145 | " | " | $3-COO-n-C_4H_9$ | |
| 1146 | " | " | $3-COO-n-C_5H_{11}$ | |
| 1147 | " | " | $3-COO-n-C_6H_{13}$ | |
| 1148 | " | " | $3-COO-n-C_8H_{17}$ | |
| 1149 | " | " | $3-COO-n-C_{10}H_{21}$ | |
| 1150 | " | " | 3- 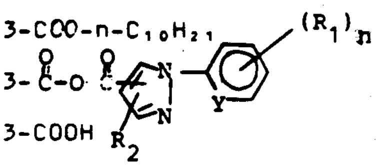 | |
| 1151 | " | " | 3-COOH | |
| 1152 | " | " | 3-COOLi | |
| 1153 | " | " | 3-COONa | |
| 1154 | " | " | 3-COOK | |
| 1155 | " | " | $3-COOCa_{1/2}$ | |
| 1156 | " | " | $3-COO-c-C_4H_7$ | |
| 1157 | " | " | $3-COO-c-C_6H_{11}$ | |
| 1158 | " | " | $3-COOCH_2-C_6H_5$ | |
| 1159 | " | " | $3-COOCH_2-(2,4-Cl_2-C_6H_3)$ | |
| 1160 | " | " | $3-COOCH_2CHCH_2$ | |
| 1161 | " | " | $3-COOC_2H_4CHCH_2$ | |
| 1162 | " | " | $3-COO-n-C_8H_{16}CHCH_2$ | |
| 1163 | " | " | $3-COO-CH_2CCH$ | |
| 1164 | " | " | $3-COO-C_2H_4-CCH$ | |
| 1165 | " | " | $3-COO-n-C_5H_{10}CCH$ | |
| 1166 | " | " | $3-COOCH_2Si(CH_3)_3$ | |
| 1167 | " | " | $3-COOC_2H_4OCH_3$ | |
| 1168 | " | " | $3-CONH_2$ | |
| 1169 | " | " | 3-CN | |
| 1170 | " | " | $3-CONHCH_3$ | |

Y=CH

| Beisp.-Nr. | $(R_1)_n$ | $R_2$ | X | $Fp/Kp_{Torr}$ [°C] |
|---|---|---|---|---|
| 1171 | 2,6,3-$(C_2H_5)_2Cl$ | 5-$CH_3$ | 3-$CONHC_2H_5$ | |
| 1172 | " | " | 3-CONH-n-$C_3H_7$ | |
| 1173 | " | " | 3-CONH-n-$C_4H_9$ | |
| 1174 | " | " | 3-CONH-n-$C_6H_{13}$ | |
| 1175 | " | " | 3-CONH-n-$C_{10}H_{21}$ | |
| 1176 | " | " | 3-CONH-i-$C_3H_7$ | |
| 1177 | " | " | 3-$CON(CH_3)_2$ | |
| 1178 | " | " | 3-$CON(CH_3)(nC_6H_{13})$ | |
| 1179 | " | " | 3-$CON(C_2H_5)_2$ | |
| 1180 | " | " | 3-CO-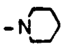 | |
| 1181 | " | " | 3-CO-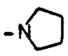 | |
| 1182 | " | " | 3-CO-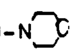 | |
| 1183 | " | " | 3-CO-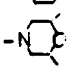 | |
| 1184 | " | " | 3-CO-NH-c-$C_6H_{11}$ | |
| 1185 | " | " | 3-CO-NH-c-$C_3H_5$ | |
| 1186 | " | " | 3-CO-$N(CH_3)(cC_6H_{11})$ | |
| 1187 | " | " | 3-COSH | |
| 1188 | " | " | 3-COSNa | |
| 1189 | " | " | 3-$COSCH_3$ | |
| 1190 | " | " | 3-$COSC_2H_5$ | |
| 1191 | " | " | 3-$COSCH_2C_6H_5$ | |
| 1192 | " | " | 3-COS-$nC_8H_{17}$ | |
| 1193 | " | " | 3-$COSC_2H_4OCH_3$ | |
| 1194 | " | " | 3-$COSCH_2CHCH_2$ | |
| 1195 | " | " | 3-$COSCH_2CCH$ | |
| 1196 | " | " | 3-COS-c-$C_6H_{11}$ | |
| 1197 | " | " | 3-$COSCH_2Si(CH_3)_3$ | |
| 1198 | " | " | 3-COS-n-$C_4H_8CH(CH_3)_2$ | |
| 1199 | " | " | 3-CO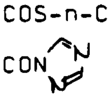 | |
| 1200 | " | " | 3-$COOC_2H_4CH(CH_3)_2$ | |

| Y=CH Beisp.-Nr. | $(R_1)_n$ | $R_2$ | X | Fp/$Kp_{Torr}$ [°C] |
|---|---|---|---|---|
| 1201 | 3-$CF_3$ | 3-$CH_3$ | 5-COOH | 164-170 |
| 1202 | 3,2,6-Cl($C_2H_5$)$_2$ | " | 5-$COOC_2H_5$ | Oel |
| 1203 | 4,2-Cl-$CF_3$-Phe | 3-$CH_6$ | 5-$COOC_2H_5$ | Oel |
| 1204 | 3-$CF_3$ | 5-C($CH_3$)$_3$ | 3-$COOC_2H_5$ | Oel |
| 1205 | 2,4-$Br_2$ | 5-C($CH_3$)$_3$ | 3-$COOC_2H_5$ | 130-132 |
| 1206 | 2,3-$Cl_2$ | 5-C($CH_3$)$_3$ | 3-$COOC_2H_5$ | 101-102 |
| 1207 | 2,6,4-$Cl_2$-$CF_3$ | 3-$CH_2CH(CH_3)_2$ | 5-$COOC_2H_5$ | Oel |
| 1208 | " | 5-$CH_2CH(CH_3)_2$ | 3-$COOC_2H_5$ | 82-84 |
| 1209 | 2,4-$Cl_2$ | 3-$CH_2CH(CH_3)_2$ | 5-$COOC_2H_5$ | Oel |
| 1210 | 2,4-$Br_2$ | 3-i-$C_3H_7$ | 5-$COOC_2H_5$ | |
| 1211 | 3-$CF_3$ | 5-$CH_2CH(CH_3)_2$ | 3-$COOC_2H_5$ | Oel |
| 1212 | 2,6,4-$Cl_2$-$CF_3$ | 5-$CH_2CH(CH_3)_2$ | 3-COOH | 191-193 |
| 1213 | 2,3-$Cl_2$-Phe | 5-CH | 3-$COOC_2H_5$ | 76-78 |
| 1214 | " | 5-$CH_2CH(CH_3)$ | 3-$COOC_2H_5$ | 91-92 |
| 1215 | 2,4-$Br_2$ | 5-$CH_2CH(CH_3)$ | 3-COOEt | Oel |
| 1216 | 2,4-$Cl_2$ | 5-$CH_3$ | 3-$COOCH_2CH(CH_3)CH_2CH_3$ | 39-45 |
| 1217 | 3-$CF_3$ | 5-$CH_3$ | 3-$COOC_2H_5$ | Oel |
| 1218 | 2,4-$Br_2$ | 5-$CH(CH_3)_2$ | 3-$COOC_2H_5$ | 72-79 |
| 1219 | 2,4-Cl-$CF_3$ | 3-$CH(CH_3)_2$ | 5-$COOC_2H_5$ | Oel |
| 1220 | " | 5-$CH(CH_3)_2$ | 3-$COOC_2H_5$ | 58-70 |
| 1221 | 2,4-$Br_2$ | 5-$CH_2CH(CH_3)_2$ | 3-$COOC_2H_5$ | 184-187 |
| 1222 | 2,4-Cl-$CF_3$ | 5-C($CH_3$)$_3$ | 3-$COOC_2H_5$ | 106-107 |
| 1223 | 2,6,4-$Cl_2$-$CF_3$ | 5-$CH_2CH(CH_3)_2$ | 3-$COO^-Li^+$ | >250 |
| 1224 | 2,3-$Cl_2$ | 5-$CH_2CH(CH_3)_2$ | 3-COOH | 209-211 |
| 1225 | 2,4-Cl-$CF_3$ | 5-$CH_2CH(CH_3)_2$ | 3-$COOC_2H_5$ | 54-58 |

| Beisp.-Nr. | $(R_1)_n$ | $R_2$ | X | Fp/Kp Torr [°C] |
|---|---|---|---|---|
| 1226 | 2,4,5-Cl, F-$CH_3$-Phe | 5-$CH_3$ | 3-$COOC_2H_5$ | 109-110 |
| 1227 | 3,4-Cl,-$CH_3$-Phe | 5-$CH_3$ | 3-$COOC_2H_5$ | 77-80 |
| 1228 | 2,4-$Cl_2$-Phe | 5-$CH_3$ | 3-COO $HN(C_2H_4OH)_3$ | 135-138 |
| 1229 | 2,4-$Cl_2$-Phe | 5-$CH_3$ | 3-$CONHC(CH_3)(CH(CH_3)_2)$-$CONH_2$ | 65-69 |
| 1230 | 2,4-$Cl_2$-Phe | 5-$CH_3$ | 3-$C(NH_2)NOH$ | 205 |
| 1231 | 2,6-$(CH_3)_2$ | 5-$CH_3$ | 3-$COOC_2H_5$ | Oel |
| 1232 | 4-F-Phe | 5-$CH_3$ | 3-$COOC_2H_5$ | Harz |
| 1233 | 4-$OCH_3$-Phe | 5-$CH_3$ | 3-$COOC_2H_5$ | Oel |
| 1234 | 2,4-Cl, $CF_3$-Phe | 3-$CH_3$ | 5-$COOC_2H_5$ | Oel |
| 1235 | 2,4-$Cl_2$ | 5-c-$C_3H_5$ | 3-$COOC_2H_5$ | 80 |
| 1236 | 2,6,4-$Cl_2$, $CF_3$-Phe | 5-c-$C_3H_5$. | 3-$COOC_2H_5$ | 105-110 |

Abkürzungen: n: geradkettig
i: iso (verzweigt)
c: cyclo

## C. Biologische Beispiele

### Beispiel 1

Weizen und Gerste wurden im Gewächshaus in Plastiktöpfen bis zum 3 bis 4 Blattstadium herangezogen und dann nacheinander mit den Safener-Verbindungen und den getesteten Herbiziden im Nachauflaufverfahren behandelt. Die Herbizide und die Verbindungen der Formel I wurden dabei in Form wäßriger Suspensionen bzw. Emulsionen mit einer Wasseraufwandmenge von umgerechnet 800 l/ha ausgebracht. 3 bis 4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde. Der Grad der Schädigung bzw. die Safenerwirkung von I wurde in % Schädigung bestimmt.

Die Ergebnisse aus Tabelle I veranschaulichen, daß die erfindungsgemäßen Verbindungen starke Herbizidschäden an den Kulturpflanzen effektiv reduzieren können.

Selbst bei starken Überdosierungen des Herbizids werden bei den Kulturpflanzen auftretende schwere Schädigungen deutlich reduziert, geringere Schäden völlig aufgehoben. Mischungen aus Herbiziden und erfindungsgemäßen Verbindungen eignen sich deshalb in vorteilhafter Weise zur selektiven Unkrautbekämpfung in Getreidekulturen.

Tabelle 1: Safenerwirkung der erfindungsgemäßen Verbindungen

| Kombination Herbizid/Safener | Dosierung (kg a.i./ha) | % Schädigung (Safenerwirkung) TA | HV |
|---|---|---|---|
| H1 | 2.0 | 80 | - |
| | 0.2 | - | 85 |
| H1 + 122 | 2.0 + 2.5 | 10 | - |
| | 0.2 + 2.5 | - | 20 |
| H1 + 148 | 2.0 + 2.5 | 50 | - |
| | 0.2 + 2.5 | - | 40 |
| H1 + 182 | 2.0 + 2.5 | 40 | - |
| | 0.2 + 2.5 | - | 35 |
| H1 + 542 | 2.0 + 2.5 | 30 | - |
| | 0.2 + 2.5 | - | 35 |
| H1 + 131 | 2.0 + 2.5 | 20 | - |
| | 0.2 + 2.5 | - | 40 |
| H1 + 191 | 2.0 + 2.5 | 20 | - |
| | 0.2 + 2.5 | - | 45 |
| H1 + 1 | 2.0 + 2.5 | 15 | - |
| | 0.2 + 2.5 | - | 45 |
| H1 + 782 | 2.0 + 2.5 | 20 | - |
| | 0.2 + 2.5 | - | 40 |
| H1 + 602 | 2.0 + 2.5 | 20 | - |
| | 0.2 + 2.5 | - | 50 |
| H1 + 1201 | 2.0 + 2.5 | 35 | - |
| | 0.2 + 2.5 | - | 50 |
| H1 + 611 | 2.0 + 2.5 | 35 | - |
| | 0.2 + 2.5 | - | 50 |
| H1 + 1202 | 2.0 + 2.5 | 50 | - |
| | 0.2 + 2.5 | - | 70 |
| H1 + 1142 | 2.0 + 2.5 | 25 | - |
| | 0.2 + 2.5 | - | 40 |
| H1 + 842 | 2.0 + 2.5 | 25 | - |
| | 0.2 + 2.5 | - | 30 |
| H1 + 902 | 2.0 + 2.5 | 50 | - |
| | 0.2 + 2.5 | - | 55 |

| Kombination Herbizid/Safener | Dosierung (kg a.i./ha) | Safenerwirkung TA | HV |
|---|---|---|---|
| H1 + 71 | 2.0 + 2.5 | 50 | - |
| | 0.2 + 2.5 | - | 65 |
| H1 + 632 | 2.0 + 2.5 | 30 | - |
| | 0.2 + 2.5 | - | 85 |
| H1 + 605 | 2.0 + 2.5 | 70 | - |
| | 0.2 + 2.5 | - | 40 |
| H1 + 722 | 2.0 + 2.5 | 20 | - |
| | 0.2 + 2.5 | - | 50 |
| H1 + 152 | 2.0 + 2.5 | 40 | - |
| | 0.2 + 2.5 | - | 85 |
| H1 + 212 | 2.0 + 2.5 | 40 | - |
| | 0.2 + 2.5 | - | 70 |
| H1 + 302 | 2.0 + 2.5 | 60 | - |
| | 0.2 + 2.5 | - | 30 |
| H1 + 362 | 2.0 + 2.5 | 20 | - |
| | 0.2 + 2.5 | - | 20 |
| H1 + 1204 | 2.0 + 2.5 | 60 | - |
| | 0.2 + 2.5 | - | 50 |
| H1 + 1205 | 2.0 + 2.5 | 60 | - |
| | 0.2 + 2.5 | - | 50 |
| H1 + 1206 | 2.0 + 2.5 | 60 | - |
| | 0.2 + 2.5 | - | 50 |
| H1 + 1207 | 2.0 + 2.5 | 55 | - |
| | 0.2 + 2.5 | - | 45 |
| H1 + 1208 | 2.0 + 2.5 | 60 | - |
| | 0.2 + 2.5 | - | 45 |
| H1 + 1209 | 2.0 + 2.5 | 70 | - |
| | 0.2 + 2.5 | - | 45 |
| H1 + 422 | 2.0 + 2.5 | 70 | - |
| | 0.2 + 2.5 | - | 50 |
| H1 + 1210 | 2.0 + 2.5 | 70 | - |
| | 0.2 + 2.5 | - | 55 |
| H1 + 1211 | 2.0 + 2.5 | 60 | - |
| | 0.2 + 2.5 | - | 50 |

| Kombination Herbizid/Safener | Dosierung (kg a.i./ha) | Safenerwirkung TA | HV |
|---|---|---|---|
| H1 + 1212 | 2.0 + 2.5 | 70 | - |
| | 0.2 + 2.5 | - | 40 |
| H1 + 1213 | 2.0 + 2.5 | 40 | - |
| | 0.2 + 2.5 | - | 30 |
| H1 + 1214 | 2.0 + 2.5 | 60 | - |
| | 0.2 + 2.5 | - | 10 |
| $H_1$ + 121 | 2,0 + 2,5 | 25 | - |
| | 0,2 + 2,5 | - | 40 |
| $H_1$ + 123 | " | 60 | - |
| | " | - | 40 |
| $H_1$ + 124 | 2,0 + 1,25 | 20 | - |
| | 0,2 + 1,25 | - | 30 |
| $H_1$ + 125 | 2,0 + 2,5 | 60 | - |
| | 0,2 + 2,5 | - | 40 |
| $H_1$ + 127 | " | 40 | - |
| | " | - | 30 |
| $H_1$ + 128 | 2,0 + 1,25 | 20 | - |
| | 0,2 + 1,25 | - | 40 |
| $H_1$ + 132 | 2,0 + 2,5 | 30 | - |
| | 0,2 + 2,5 | - | 30 |
| $H_1$ + 133 | 2,0 + 1,25 | 20 | - |
| | 0,2 + 1,25 | - | 30 |
| $H_1$ + 135 | 2,0 + 2,5 | 30 | - |
| | 0,2 + 2,5 | - | 30 |
| $H_1$ + 137 | 2,0 + 1,25 | 40 | - |
| | 0,2 + 1,25 | - | 50 |
| $H_1$ + 138 | " | 10 | - |
| | " | - | 20 |
| $H_1$ + 140 | " | 20 | - |
| | " | - | 40 |
| $H_1$ + 143 | " | 15 | - |
| | " | - | 60 |

| Produkt (Herbizid/Safener) | Dosierung (kg a.i. /ha) | Safenerwirkung | |
|---|---|---|---|
| | | TA | HV |
| $H_1$ + 146 | 2,0 + 1,25 | 40 | - |
| | 0,2 + 1,25 | - | 70 |
| $H_1$ + 147 | " | 20 | - |
| | " | - | 20 |
| $H_1$ + 149 | " | 35 | - |
| | " | - | 40 |
| $H_1$ + 150 | " | 30 | - |
| | " | - | 80 |
| $H_1$ + 153 | " | 10 | - |
| | " | - | 30 |
| $H_1$ + 157 | " | 50 | - |
| | " | - | 75 |
| $H_1$ + 159 | " | 20 | - |
| | " | - | 20 |
| $H_1$ + 160 | " | 50 | - |
| | " | - | 60 |
| $H_1$ + 162 | " | 30 | - |
| | " | - | 80 |
| $H_1$ + 164 | " | 10 | - |
| | " | - | 70 |
| $H_1$ + 171 | " | 20 | - |
| | " | - | 75 |
| $H_1$ + 242 | " | 20 | - |
| | " | - | 30 |
| $H_1$ + 251 | " | 20 | - |
| | " | - | 20 |
| $H_1$ + 301 | " | 20 | - |
| | " | - | 30 |
| $H_1$ + 303 | " | 10 | - |
| | " | - | 20 |
| $H_1$ + 311 | " | 30 | - |
| | " | - | 30 |

| Produkt (Herbizid/Safener) | Dosierung (kg a.i. /ha) | Safenerwirkung TA | HV |
|---|---|---|---|
| $H_1$ + 361 | 2,0 + 1,25 | 15 | - |
| | 0,2 + 1,25 | - | 20 |
| $H_1$ + 391 | " | 25 | - |
| | " | - | 50 |
| $H_1$ + 392 | " | 20 | - |
| | " | - | 70 |
| $H_1$ + 482 | " | 20 | - |
| | " | - | 40 |
| $H_1$ + 491 | " | 20 | - |
| | " | - | 40 |
| $H_1$ + 511 | " | 30 | - |
| | " | - | 85 |
| $H_1$ + 692 | " | 30 | - |
| | " | - | 40 |
| $H_1$ + 1022 | " | 30 | - |
| | " | - | 70 |
| $H_1$ + 1218 | 2,0 + 2,5 | 30 | - |
| | 0,2 + 2,5 | - | 20 |
| $H_1$ + 1219 | " | 35 | - |
| | " | - | 50 |
| $H_1$ + 1220 | " | 30 | - |
| | " | - | 20 |
| $H_1$ + 1221 | " | 30 | - |
| | " | - | 20 |
| $H_1$ + 1222 | " | 15 | - |
| | " | - | 30 |
| $H_1$ + 1223 | " | 20 | - |
| | " | - | 60 |
| $H_1$ + 1224 | " | 20 | - |
| | " | - | 60 |
| $H_1$ + 1225 | " | 50 | - |
| | " | - | 30 |

| Produkt (Herbizid/Safener) | Dosierung (kg a.i. /ha) | Safenerwirkung TA | HV |
|---|---|---|---|
| $H_1$ + 1226 | 2,0 + 1,25 | 30 | - |
| | 0,2 + 1,25 | - | 70 |
| $H_1$ + 1227 | " | 50 | - |
| | " | - | 80 |
| $H_1$ + 1228 | " | 40 | - |
| | " | - | 70 |
| $H_1$ + 1229 | " | 30 | - |
| | " | - | 60 |
| $H_1$ + 1230 | " | 50 | - |
| | " | - | 80 |
| $H_1$ + 1231 | " | 40 | - |
| | " | - | 75 |
| $H_1$ + 1233 | " | 40 | - |
| | " | - | 75 |
| $H_1$ + 1235 | " | 20 | - |
| | " | - | 40 |
| $H_1$ + 1236 | " | 20 | - |
| | " | - | 60 |

Abkürzungen: TA = Triticum aestivum (Weizen)
HV = Hordeum vulgare (Gerste)
a.i. = Aktivsubstanz
H1 = Fenoxaprop-ethyl

## Patentansprüche

## Patentansprüche für folgende Vertragsstaaten : AT, BE, CH, DE, FR, GB, IT, LI

**1.** Pflanzenbehandlungsmittel, dadurch gekennzeichnet, daß sie eine Verbindung der Formel I

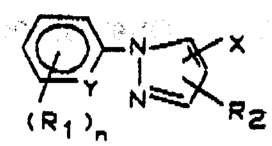

(I),

worin

Y C-H oder N,

$R_1$ unabhängig voneinander $(C_1$-$C_4)$-Alkyl, $(C_1$-$C_4)$-Haloalkyl, $(C_1$-$C_4)$-Alkoxy, $(C_1$-$C_4)$-Haloalkoxy oder Halogen,

$R_2$ $(C_1$-$C_{12})$-Alkyl oder $(C_3$-$C_7)$-Cycloalkyl,

X $COOR_3$, $CON(R_4)_2$, $COSR_3$, CN,

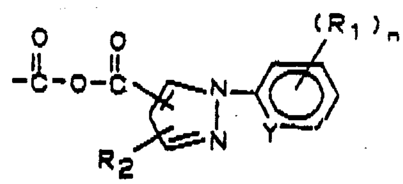

$R_3$ Alkali- oder Erdalkalimetall, Wasserstoff, $(C_1-C_{10})$-Alkyl, $(C_3-C_{20})$-Alkenyl, $(C_3-C_{10})$-Alkinyl, $(C_3-C_7)$-Cycloalkyl, Phenyl-$(C_1-C_4)$-alkyl, wobei Phenyl durch Halogen substituiert sein kann, Tris-[$(C_1-C_4)$-alkyl]-silyl-$(C_1-C_4)$-alkyl, $(C_1-C_4)$-Alkoxy-$(C_1-C_4)$-alkyl,

$R_4$ unabhängig voneinander H, $(C_1-C_{10})$-Alkyl, $(C_3-C_7)$-Cycloalkyl, das substituiert sein kann, oder 2 Reste $R_4$ bilden zusammen mit dem sie verknüpfenden N-Atom einen 4- bis 7-gliedrigen heterocyclischen Ring und

n 1 bis 3

bedeuten, als Safener zum Schutz von Kulturpflanzen gegen phytotoxische Nebenwirkungen Von Herbiziden in Kombination mit einem Herbizid enthalten.

**2.** Mittel gemäß Anspruch 1, dadurch gekennzeichnet, daß in Formel I Y = CH, $R_1$ = Halogen, $(C_1-C_4)$-Haloalkyl, $R_2$ = $(C_1-C_6)$-Alkyl, X = $COOR_3$, $R_3$ = H oder $(C_1-C_6)$Alkyl und n = 1 oder 2 bedeuten.

**3.** Mittel gemäß Anspruch 1, dadurch gekennzeichnet, daß Y = CH, $R_1$ = Cl, Br, oder $CF_3$, $R_2$ = $(C_1-C_4)$-Alkyl, X = $COOR_3$, $R_3$ = $(C_1-C_4)$-Alkyl und n = 2 bedeuten.

**4.** Mittel gemäß einem oder mehreren der Ansprüche 1 bis 3, dadurch gekennzeichnet, daß als Herbizid eine Verbindung vom Typ der Phenoxyphenoxy- oder Heteroaryloxyphenoxycarbonsäure-$(C_1-C_4)$-alkyl-, $(C_2-C_4)$-alkenyl- oder $(C_3-C_4)$-alkinylester eingesetzt wird.

**5.** Mittel gemäß einem oder mehreren der Ansprüche 1 bis 4, dadurch gekennzeichnet, daß als Herbizid 2-(4-(6-Chlorbenzoxazol-2-yl-oxy)-phenoxy)-propionsäureethylester oder 2-(4-(6-Chlorbenzthiazol-2-yl-oxy)-phenoxy)-propionsäureethylester eingesetzt wird.

**6.** Mittel gemäß Anspruch 4, dadurch gekennzeichnet, daß als Herbizid die Verbindung 2-(4-(5-Chlor-3-fluor-pyridyl-2-oxy)-phenoxy)-propionsäurepropargylester eingesetzt wird.

**7.** Mittel gemäß einem der Ansprüche 1 bis 3, dadurch gekennzeichnet, daß als Herbizid die Verbindung 2-(N-Ethoxypropionamidoyl)-5-mesityl-3-hydroxy-2-cyclohexen-1-on eingesetzt wird.

**8.** Mittel gemäß einem der Ansprüche 1 bis 7, dadurch gekennzeichnet, daß das Verhältnis Safener zu Herbizid 1:10 bis 10:1 beträgt.

**9.** Mittel gemäß Anspruch 8, dadurch gekennzeichnet, daß das Verhältnis Safener zu Herbizid 2:1 bis 1:10 beträgt.

**10.** Verbindung der Formel I gemäß Anspruch 1, worin Y = CH, $R_1$ = 2,4-$Cl_2$, $R_2$ = isopropyl, X = $COOR_3$ und $R_3$ = $(C_1-C_{10})$-Alkyl bedeuten.

**11.** Verbindung gemäß Anspruch 10, Worin Y = CH, $R_1$ = 2,4-$Cl_2$, $R_2$ = 5-Isopropyl und X = 3-$COOC_2H_5$ bedeuten.

**12.** Verfahren zur Minderung der Phytotoxizität von Herbiziden gegenüber Kulturpflanzen, dadurch gekennzeichnet, daß man die Pflanzen, Pflanzensamen oder Anbauflächen mit einer wirksamen Menge einer Verbindung der Formel I gemäß einem der Ansprüche 1, 2, 3, 10 und 11 vor, nach oder gleichzeitig mit dem Herbizid behandelt.

**13.** Verwendung von Verbindungen der Formel I gemäß einem der Ansprüche 1, 2, 3, 10 und 11 zur Minderung der Phytotoxizität von Herbiziden gegenüber Kulturpflanzen.

**14.** Verwendung nach Anspruch 13, dadurch gekennzeichnet, daß als Herbizid Fenoxaprop-ethyl eingesetzt wird.

**Patentansprüche für folgenden Vertragsstaat : ES**

**1.** Verwendung von Verbindungen der Formel I

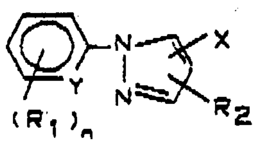

(I),

worin

Y C-H oder N,

$R_1$ unabhängig voneinander $(C_1\text{-}C_4)$-Alkyl, $(C_1\text{-}C_4)$-Haloalkyl, $(C_1\text{-}C_4)$-Alkoxy, $(C_1\text{-}C_4)$-Haloalkoxy oder Halogen,

$R_2$ $(C_1\text{-}C_{12})$-Alkyl oder $(C_3\text{-}C_7)$-Cycloalkyl,

X $COOR_3$, $CON(R_4)_2$, $COSR_3$, CN,

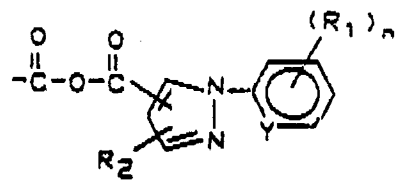

$R_3$ Alkali- oder Erdalkalimetall, Wasserstoff, $(C_1\text{-}C_{10})$-Alkyl, $(C_3\text{-}C_{20})$-Alkenyl, $(C_3\text{-}C_{10})$-Alkinyl, $(C_3\text{-}C_7)$-Cycloalkyl, Phenyl-$(C_1\text{-}C_4)$-alkyl, wobei Phenyl durch Halogen substituiert sein kann, Tris-[$(C_1\text{-}C_4)$-alkyl]-silyl-$(C_1\text{-}C_4)$-alkyl, $(C_1\text{-}C_4)$-Alkoxy-$(C_1\text{-}C_4)$-alkyl,

$R_4$ unabhängig voneinander H, $(C_1\text{-}C_{10})$-Alkyl, $(C_3\text{-}C_7)$-Cycloalkyl, das substituiert sein kann, oder 2 Reste $R_4$ bilden zusammen mit dem sie verknüpfenden N-Atom einen 4- bis 7-gliedrigen heterocyclischen Ring und

n 1 bis 3 bedeuten, als Safener zum Schutz von Kulturpflanzen gegen phytotoxische Nebenwirkungen von Herbiziden.

**2.** Verwendung gemäß Anspruch 1, dadurch gekennzeichnet, daß in Formel I Y = CH, $R_1$ = Halogen, $(C_1\text{-}C_4)$-Haloalkyl, $R_2$ = $(C_1\text{-}C_6)$-Alkyl, X = $COOR_3$, $R_3$ = H oder $(C_1\text{-}C_4)$-Alkyl und n = 1 oder 2 bedeuten.

**3.** Verwendung gemäß Anspruch 1, dadurch gekennzeichnet, daß Y = CH, $R_1$ = Cl, Br, oder $CF_3$, $R_2$ = $(C_1\text{-}C_4)$-Alkyl, X = $COOR_3$, $R_3$ = $(C_1\text{-}C_4)$-Alkyl und n = 2 bedeuten.

**4.** Verwendung gemäß einem oder mehreren der Ansprüche 1 bis 3, dadurch gekennzeichnet, daß als Herbizid eine Verbindung vom Typ der Phenoxyphenoxy- oder Heteroaryloxyphenoxycarbonsäure-$(C_1\text{-}C_4)$-alkyl-, $(C_2\text{-}C_4)$-alkenyl- oder $(C_3\text{-}C_4)$-alkinylester eingesetzt wird.

**5.** Verwendung gemäß einem oder mehreren der Ansprüche 1 bis 4, dadurch gekennzeichnet, daß als Herbizid 2-(4-(6-Chlorbenzoxazol-2-yl-oxy)-phenoxy)-propionsäureethylester oder 2-(4-(6-Chlorbenzthiazol-2-yl-oxy)-phenoxy)-propionsäureethylester eingesetzt wird.

**6.** Verwendung gemäß Anspruch 4, dadurch gekennzeichnet, daß als Herbizid die Verbindung 2-(4-(5-Chlor-3-fluor-pyridyl-2-oxy)-phenoxy)-propionsäurepropargylester eingesetzt wird.

**7.** Verwendung gemäß einem der Ansprüche 1 bis 3, dadurch gekennzeichnet, daß als Herbizid die Verbindung 2-(N-Ethoxypropionamidoyl)-5-mesityl-3-hydroxy-2-cyclohexen-1-on eingesetzt wird.

**8.** Verwendung gemäß einem der Ansprüche 1 bis 7, dadurch gekennzeichnet, daß das Verhältnis Safener zu Herbizid 1:10 bis 10:1 beträgt.

**9.** Verwendung gemäß Anspruch 8, dadurch gekennzeichnet, daß das Verhältnis Safener zu Herbizid 2:1 bis 1:10 beträgt.

**10.** Verwendung nach einem der Ansprüche 1 bis 9, dadurch gekennzeichnet, daß in Formel I Y = CH, $R_1$ = 2,4-$Cl_2$, $R_2$ = isopropyl, X = $COOR_3$ und $R_3$ = ($C_1$-$C_{10}$)-Alkyl bedeuten.

**11.** Verwendung gemäß Anspruch 10, dadurch gekennzeichnet, daß Y = CH, $R_1$ = 2,4-$Cl_2$, $R_2$ = 5-Isopropyl und X = 3-$COOC_2H_5$ bedeuten.

**12.** Verfahren zur Minderung der Phytotoxizität von Herbiziden gegenüber Kulturpflanzen, dadurch gekennzeichnet, daß man die Pflanzen, Pflanzensamen oder Anbauflächen mit einer wirksamen Menge einer Verbindung der Formel I gemäß einem der Ansprüche 1, 2, 3, 10 und 11 vor, nach oder gleichzeitig mit dem Herbizid behandelt.

**Claims**
**Claims for the following Contracting States : AT, BE, CH, DE, FR, GB, IT, LI**

**1.** A plant treatment agent which contains a compound of the formula I

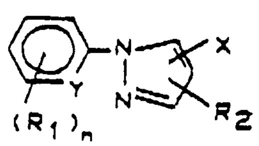

(I)

wherein

Y denotes C-H or N,
the symbols $R_1$ independently of one another denote ($C_1$-$C_4$)-alkyl, ($C_1$-$C_4$)-haloalkyl, ($C_1$-$C_4$)-alkoxy, ($C_1$-$C_4$)-haloalkoxy or halogen,
$R_2$ denotes ($C_1$-$C_{12}$)-alkyl or ($C_3$-$C_7$)-cycloalkyl,
x denotes $COOR_3$, $CON(R_4)_2$, $COSR_3$, CN or

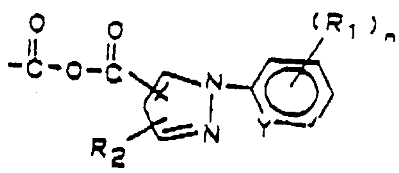

$R_3$ denotes an alkali metal or alkaline earth metal, hydrogen, ($C_1$-$C_{10}$)-alkyl, ($C_3$-$C_{20}$)-alkenyl, ($C_3$-$C_{10}$)-alkynyl, ($C_3$-$C_7$)-cycloalkyl or phenyl-($C_1$-$C_4$)-alkyl, it being possible for phenyl to be substituted by halogen, or tris-[($C_1$-$C_4$)-alkyl]-silyl-($C_1$-$C_4$)-alkyl or ($C_1$-$C_4$)-alkoxy-($C_1$-$C_4$)-alkyl,
the symbols $R_4$ independently of one another denote H, ($C_1$-$C_{10}$)-alkyl, or ($C_3$-$C_7$)-cycloalkyl, which can be substituted, or 2 radicals $R_4$ together with the N atom linking them form a 4- to 7-membered heterocyclic ring and
n denotes 1 to 3,

as a safener for protecting crop plants from the phytotoxic side-effects of herbicides, in combination with a herbicide.

**2.** An agent as claimed in claim 1, wherein, in formula I, Y denotes CH, $R_1$ denotes halogen or ($C_1$-$C_4$)-haloalkyl, $R_2$ denotes ($C_1$-$C_6$)-alkyl, X denotes $COOR_3$, $R_3$ denotes H or ($C_1$-$C_6$)-alkyl and n denotes 1 or 2.

**3.** An agent as claimed in claim 1, wherein Y denotes CH, $R_1$ denotes Cl, Br or $CF_3$, $R_2$ denotes $(C_1\text{-}C_4)$-alkyl, X denotes $COOR_3$, $R_3$ denotes $(C_1\text{-}C_4)$-alkyl and n denotes 2.

**4.** An agent as claimed in one or more of claims 1 to 3, wherein a compound of the $(C_1\text{-}C_4)$-alkyl, $(C_2\text{-}C_4)$-alkenyl or $(C_3\text{-}C_4)$-alkynyl phenoxyphenoxy- or heteroaryloxy-phenorycarboxylate type is used as the herbicide.

**5.** An agent as claimed in one or more of claims 1 to 4, wherein ethyl 2-(4-(6-chlorobenzoxazol-2-yloxy)-phenoxy)propionate or ethyl 2-(4-(6-chlorobenzothiazol-2-yloxy)phenoxy)propionate is used as the herbicide.

**6.** An agent as claimed in claim 4, wherein the compound propargyl 2-(4-(5-chloro-3-fluoropyridyl-2-oxy)-phenoxy)propionate is used as the herbicide.

**7.** An agent as claimed in any one of claims 1 to 3, wherein the compound 2-(N-ethoxypropionamidoyl)-5-mesityl-3-hydroxy-2-cyclohexen-1-one is used as the herbicide.

**8.** An agent as claimed in any one of claims 1 to 7, wherein the ratio of safener to herbicide is 1:10 to 10:1.

**9.** An agent as claimed in claim 8, wherein the ratio of safener to herbicide is 2:1 to 1:10.

**10.** A compound of the formula I as in claim 1, in which Y denotes CH, $R_1$ denotes 2,4-$Cl_2$, $R_2$ denotes isopropyl, X denotes $COOR_3$ and $R_3$ denotes $(C_1\text{-}C_{10})$-alkyl.

**11.** A compound as claimed in claim 1, wherein Y denotes CH, $R_1$ denotes 2,4-$Cl_2$, $R_2$ denotes 5-isopropyl and X denotes 3-$COOC_2H_5$.

**12.** A method of reducing the phytotoxicity of herbicides toward crop plants, which comprises treating the plants, plant seeds or cultivation areas with an effective amount of a compound of the formula I as in any one of claims 1, 2 and 3 or as claimed in either of claims 10 and 11 before, after or at the same time as the herbicide.

**13.** The use of a compound of the formula I as in any one of claims 1, 2 and 3 or as claimed in either of claims 10 and 11 for reducing the phytotoxicity of herbicides toward crop plants.

**14.** The use as claimed in claim 13, wherein fenoxaprop-ethyl is used as the herbicide.

**Claims for the following Contracting State : ES**

**1.** The use of a compound of the formula I

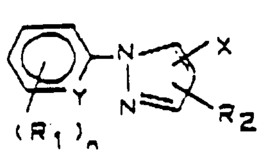

(I)

wherein

Y denotes C-H or N,

the symbols $R_1$ independently of one another denote $(C_1\text{-}C_4)$-alkyl, $(C_1\text{-}C_4)$-haloalkyl, $(C_1\text{-}C_4)$-alkoxy, $(C_1\text{-}C_4)$-haloalkoxy or halogen,

$R_2$ denotes $(C_1\text{-}C_{12})$-alkyl or $(C_3\text{-}C_7)$-cycloalkyl,

X denotes $COOR_3$, $CON(R_4)_2$, $COSR_3$, CN or

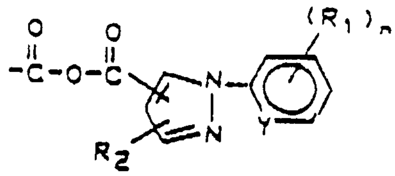

$R_3$ denotes an alkali metal or alkaline earth metal, hydrogen, ($C_1$-$C_{10}$)-alkyl, ($C_3$-$C_{20}$)-alkenyl, ($C_3$-$C_{10}$)-alkynyl, ($C_3$-$C_7$)-cycloalkyl or phenyl-($C_1$-$C_4$)-alkyl, it being possible for phenyl to be substituted by halogen, or tris-[($C_1$-$C_4$)]-alkyl-silyl-($C_1$-$C_4$)-alkyl or ($C_1$-$C_4$)-alkoxy-($C_1$-$C_4$)-alkyl,
the symbols $R_4$ independently of one another denote H, ($C_1$-$C_{10}$)-alkyl, or ($C_3$-$C_7$)-cycloalkyl, which can be substituted, or 2 radicals $R_4$ together with the N atom linking them form a 4- to 7-membered heterocyclic ring and

n denotes 1 to 3,

as a safener for protecting crop plants from the phytotoxic side-effects of herbicides.

**2.** The use as claimed in claim 1, wherein, in formula I, Y denotes CH, $R_1$ denotes halogen or ($C_1$-$C_4$)-haloalkyl, $R_2$ denotes ($C_1$-$C_6$)-alkyl, X denotes $COOR_3$, $R_3$ denotes H or ($C_1$-$C_6$)-alkyl and n denotes 1 or 2.

**3.** The use as claimed in claim 1, wherein Y denotes CH, $R_1$ denotes Cl, Br or $CF_3$, $R_2$ denotes ($C_1$-$C_4$)-alkyl, X denotes $COOR_3$, $R_3$ denotes ($C_1$-$C_4$)-alkyl and n denotes 2.

**4.** The use as claimed in one or more of claims 1 to 3, wherein a compound of the ($C_1$-$C_4$)-alkyl, ($C_2$-$C_4$)-alkenyl or ($C_3$-$C_4$)-alkynyl phenoxyphenoxy- or heteroaryloxy-phenoxycarboxylate type is used as the herbicide.

**5.** The use as claimed in one or more of claims 1 to 4, wherein ethyl 2-(4-(6-chlorobenzoxazol-2-yloxy)-phenoxy)propionate or ethyl 2-(4-(6-chlorobenzothiazol-2-yloxy)phenoxy)propionate is used as the herbicide.

**6.** An agent as claimed in claim 4, wherein the compound propargyl 2-(4-(5-chloro-3-fluoropyridyl-2-oxy)-phenoxy)propionate is used as the herbicide.

**7.** The use as claimed in any one of claims 1 to 3, wherein the compound 2-(N-ethoxypropionamidoyl)-5-mesityl-3-hydroxy-2-cyclohexen-1-one is used as the herbicide.

**8.** The use as claimed in any one of claims 1 to 7, wherein the ratio of safener to herbicide is 1:10 to 10:1.

**9.** The use as claimed in claim 8, wherein the ratio of safener to herbicide is 2:1 to 1:10.

**10.** The use as claimed in any one of claims 1 to 9, wherein, in formula I, Y denotes CH, $R_1$ denotes 2,4-$Cl_2$, $R_2$ denotes isopropyl, X denotes $COOR_3$ and $R_3$ denotes ($C_1$-$C_{10}$)-alkyl.

**11.** The use as claimed in claim 10, wherein Y denotes CH, $R_1$ denotes 2,4-$Cl_2$, $R_2$ denotes 5-isopropyl and X denotes 3-$COOC_2H_5$.

**12.** A method of reducing the phytotoxicity of herbicides toward crop plants, which comprises treating the plants, plant seeds or cultivation areas with an effective amount of a compound of the formula I as in any one of claims 1, 2, 3, 10 and 11 before, after or at the same time as the herbicide.

**Revendications**

**Revendications pour les Etats contractants suivants : AT, BE, CH, DE, FR, GB, IT, LI**

1. Produits pour le traitement des plantes, caractérisés en ce qu'ils contiennent un composé de formule I

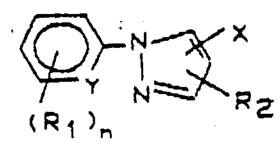

(I),

dans laquelle

Y est C-H ou N,

les radicaux $R_1$, indépendamment les uns des autres, sont chacun un radical alkyle en $C_1$-$C_4$, halogénoalkyle en $C_1$-$C_4$, alcoxy en $C_1$-$C_4$, halogénoalcoxy en $C_1$-$C_4$ ou halogéno,

$R_2$ est un radical alkyle en $C_1$-$C_{12}$ ou cycloalkyle en $C_3$-$C_7$,

X est $COOR_3$, $CON(R_4)_2$, $COSR_3$, CN,

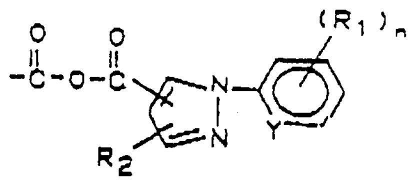

$R_3$ est un métal alcalin ou alcalino-terreux, un hydrogène ou un radical alkyle en $C_1$-$C_{10}$, alcényle en $C_3$-$C_{20}$, alcynyle en $C_3$-$C_{10}$, cycloalkyle en $C_3$-$C_7$, phényl-(alkyle en $C_1$-$C_4$), où le fragment phényle peut être substitué par des halogènes, tris-(alkyle en $C_1$-$C_4$)-silyl-(alkyle en $C_1$-$C_4$), (alcoxy en $C_1$-$C_4$)-(alkyle en $C_1$-$C_4$),

les radicaux $R_4$, indépendamment l'un de l'autre, sont chacun H ou un radical alkyle en $C_1$-$C_{10}$, cycloalkyle en $C_3$-$C_7$ pouvant être substitué, ou encore deux radicaux $R_4$ forment avec l'atome d'azote qui les relie un noyau hétérocyclique à 4-7 chaînons, et

n vaut de 1 à 3

en tant qu'antidote pour protéger les plantes de culture contre les effets secondaires phytotoxiques des herbicides, en combinaison avec un herbicide.

2. Produits selon la revendication 1, caractérisés en ce que, dans la formule I, Y est CH, $R_1$ est un halogène ou un radical halogénoalkyle en $C_1$-$C_4$, $R_2$ est un radical alkyle en $C_1$-$C_6$, X est $COOR_3$, $R_3$ est H ou un radical alkyle en $C_1$-$C_6$ et n vaut 1 ou 2.

3. Produits selon la revendication 1, caractérisés en ce que Y est $CH_3$, $R_1$ est Cl, Br ou $CF_3$, $R_2$ est un radical alkyle en $C_1$-$C_4$, X est $COOR_3$, $R_3$ est un radical alkyle en $C_1$-$C_4$ et n vaut 2.

4. Produits selon l'une ou plusieurs des revendications 1 à 3, caractérisé en ce qu'on utilise comme herbicide un composé du type phénoxyphénoxy- ou hétéroaryloxyphénoxycarboxylated'alkyle en $C_1$-$C_4$, d'alcényle en $C_2$-$C_4$ ou d'alcényle en $C_3$-$C_4$.

5. Produits selon l'une ou plusieurs des revendications 1 à 4, caractérisés en ce qu'on utilise comme herbicide le ((chloro-6 benzoxazolyl-2 oxy)-4 phénoxy)-2 propionate d'éthyle ou le ((chloro-6 benzothiazolyl-2 oxy)-4 phénoxy)-2 propionate d'éthyle.

6. Produits selon la revendication 4, caractérisés en ce qu'on utilise comme herbicide le composé (-(chloro5 fluoro-3 pyridyloxy-2)-4 phénoxy)-2 propionate de propargyle.

7. Produits selon l'une des revendications 1 à 3, caractérisés en ce qu'on utilise comme herbicide le composé N-éthoxypropionamidoyl-2 mésityl-5 hydroxy-3 cyclohexène-2 one-1.

**8.** Produits selon l'une des revendications 1 à 7, caractérisés en ce que le rapport de l'antidote à l'herbicide est de 1:10 à 10:1.

**9.** Produits selon la revendication 8, caractérisés en ce que le rapport de l'antidote à l'herbicide est de 2:1 à 1:10.

**10.** Composé de formule I selon la revendication 1, dans lequel Y est CH, $R_1$ est $Cl_2$-2,4, $R_2$ est le radical isopropyle, X est $COOR_3$ et $R_3$ est un radical alkyle en $C_1$-$C_{10}$.

**11.** Composé selon la revendication 10, dans lequel Y est CH, $R_1$ est $Cl_2$-2,4, $R_2$ est le radical isopropyl-5 et X est $COOC_2H_5$-3.

**12.** Procédé pour diminuer la phytotoxicité des herbicides vis-à-vis des plantes de culture, caractérisé en ce qu'on traite les végétaux, les semences ou les surfaces cultivées avec une quantité efficace d'un composé de formule I selon l'une des revendications 1, 2, 3, 10 ou 11, avant, pendant ou après la levée.

**13.** Utilisation de composés de formule I selon l'une des revendications 1, 2, 3, 10 ou 11, pour diminuer la phytotoxicité des herbicides vis-à-vis des plantes de culture.

**14.** Utilisation selon la revendication 13, caractérisée en ce qu'on utilise comme herbicide du fénoxapropéthyl.

**Revendications pour l'Etat contractant suivant : ES**

**1.** Utilisation de composés de formule I

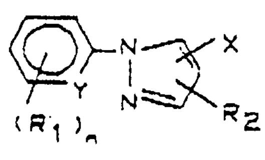

(I),

dans laquelle

Y est C-H ou N,

les radicaux $R_1$, indépendamment les uns des autres, sont chacun un radical alkyle en $C_1$-$C_4$, halogénoalkyle en $C_1$-$C_4$, alcoxy en $C_1$-$C_4$, halogénoalcoxy en $C_1$-$C_4$ ou halogéno,

$R_2$ est un radical alkyle en $C_1$-$C_{12}$ ou cycloalkyle en $C_3$-$C_7$,

X est $COOR_3$, $CON(R_4)_2$, $COSR_3$, CN,

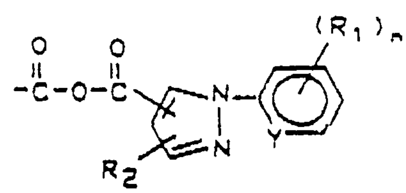

$R_3$ est un métal alcalin ou alcalino-terreux, un hydrogène ou un radical alkyle en $C_1$-$C_{10}$, alcényle en $C_3$-$C_{20}$, alcynyle en $C_3$-$C_{10}$, cycloalkyle en $C_3$-$C_7$, phényl-(alkyle en $C_1$-$C_4$), où le fragment phényle peut être substitué par des halogènes, tris-(alkyle en $C_1$-$C_4$)-silyl-(alkyle en $C_1$-$C_4$), (alcoxy en $C_1$-$C_4$)-(alkyle en $C_1$-$C_4$),

les radicaux $R_4$, indépendamment l'un de l'autre, sont chacun H ou un radical alkyle en $C_1$-$C_{10}$, cycloalkyle en $C_3$-$C_7$ pouvant être substitué, ou encore deux radicaux $R_4$ forment avec l'atome d'azote qui les relie un noyau hétérocyclique à 4-7 chaînons, et

n vaut de 1 à 3

en tant qu'antidote pour protéger les plantes de culture contre les effets secondaires phytotoxiques des herbicides, en combinaison avec un herbicide.

2. Utilisation selon la revendication 1, caractérisée en ce que, dans la formule I, Y est CH, $R_1$ est un halogène ou un radical halogénoalkyle en $C_1$-$C_4$, $R_2$ est un radical alkyle en $C_1$-$C_6$, X est $COOR_3$, $R_3$ est H ou un radical alkyle en $C_1$-$C_6$ et n vaut 1 ou 2.

3. Utilisation selon la revendication 1, caractérisée en ce que Y est $CH_3$, $R_1$ est Cl, Br ou $CF_3$, $R_2$ est un radical alkyle en $C_1$-$C_4$, X est $COOR_3$, $R_3$ est un radical alkyle en $C_1$-$C_4$ et n vaut 2.

4. Utilisation selon l'une ou plusieurs des revendications 1 à 3, caractérisé en ce qu'on utilise comme herbicide un composé du type phénoxyphénoxy- ou hétéroaryloxyphénoxycarboxylated'alkyle en $C_1$-$C_4$, d'alcényle en $C_2$-$C_4$ ou d'alcényle en $C_3$-$C_4$.

5. Utilisation selon l'une ou plusieurs des revendications 1 à 4, caractérisée en ce qu'on utilise comme herbicide le ((chloro-6 benzoxazolyl-2 oxy)-4 phénoxy)-2 propionate d'éthyle ou le ((chloro-6 benzothiazolyl-2 oxy)-4 phénoxy)-2 propionate d'éthyle.

6. Utilisation selon la revendication 4, caractérisée en ce qu'on utilise comme herbicide le composé (-(chloro-5 fluoro-3 pyridyloxy-2)-4 phénoxy)-2 propionate de propargyle.

7. Utilisation selon l'une des revendications 1 à 3, caractérisée en ce qu'on utilise comme herbicide le composé N-éthoxypropionamidoyl-2 mésityl-5 hydroxy-3 cyclohexène-2 one-1.

8. Utilisation selon l'une des revendications 1 à 7, caractérisée en ce que le rapport de l'antidote à l'herbicide est de 1:10 à 10:1.

9. Utilisation selon la revendication 8, caractérisée en ce que le rapport de l'antidote à l'herbicide est de 2:1 à 1:10.

10. Utilisation selon l'une des revendications 1 à 9, caractérisée en ce que, dans la formule I, Y est CH, $R_1$ est $Cl_2$-2,4, $R_2$ est le radical isopropyle, X est $COOR_3$ et $R_3$ est un radical alkyle en $C_1$-$C_{10}$.

11. Utilisation selon la revendication 10, caractérisée en ce que Y est CH, $R_1$ est $Cl_2$-2,4, $R_2$ est le radical isopropyl-5 et X est $COOC_2H_5$-3.

12. Procédé pour diminuer la phytotoxicité des herbicides vis-à-vis des plantes de culture, caractérisé en ce qu'on traite les végétaux, les semences ou les surfaces cultivées avec une quantité efficace d'un composé de formule I selon l'une des revendications 1, 2, 3, 10 ou 11, avant, pendant ou après la levée.